(19) Europäisches Patentamt
European Patent Office
Office européen des brevets

(11) EP 0 467 839 B1

(12) **EUROPEAN PATENT SPECIFICATION**

(45) Date of publication and mention of the grant of the patent:
**21.08.1996 Bulletin 1996/34**

(51) Int Cl.[6]: **C12N 15/62**, C12P 21/00, C12N 1/19

(21) Application number: **91810543.8**

(22) Date of filing: **09.07.1991**

(54) **In vitro processing of fusion proteins**

In vitro-Behandlung von Fusionsproteinen

Traitement in vitro de protéine fusion

(84) Designated Contracting States:
**AT BE CH DE DK ES FR GB GR IT LI LU NL SE**

(30) Priority: **18.07.1990 GB 9015825**

(43) Date of publication of application:
**22.01.1992 Bulletin 1992/04**

(83) **Declaration under Rule 28(4) EPC (expert solution)**

(73) Proprietor: **CIBA-GEIGY AG**
**4002 Basel (CH)**

(72) Inventors:
- **Heim, Jutta, Dr.**
  **CH-4433 Ramlinsburg (CH)**
- **Seeboth, Peter, Dr.**
  **W-7854 Inzlingen (DE)**
- **Takabayashi, Kenji, Dr.**
  **CH-4123 Allschwil (CH)**

(56) References cited:
**EP-A- 0 252 854** **EP-A- 0 301 669**

- **FEBS LETTERS, vol. 218, no. 1, June 1987, Amsterdam (NL); J.C. WAGNER et al., pp. 31-34**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 467 839 B1

## Description

The present invention relates to a method for the production of mature proteins by in vitro processing of fusion proteins, and to means required for the preparation of such fusion proteins.

The production of pharmaceutically applicable or enzymatically active proteins is a key area in the rapidly developing biotechnology industry. Since the beginning of the era of recombinant DNA technology a great number of valuable heterologous proteins have been expressed in prokaryotic and eukaryotic host cells which had been transformed with suitable expression vectors containing DNA sequences coding for said proteins. In many cases, however, great difficulties are encountered in producing sufficient amounts of pure products. It is frequently observed, especially in the case of low molecular weight proteins, that the product is impure due to the presence of varying amounts of degraded, in particular C-terminal degraded, byproducts lowering the yield and rendering the purification a difficult task. In other cases the overall yield in the desired protein is unsatisfactory and cannot significantly be raised by modification in the process (e.g. choice of particular vectors and host strains, condition of cultivation). Furthermore, protein purification is not a trivial task. Frequently, the primary isolation mixture contains only an utmost minor amount of the desired protein while host-specific proteins are dominating. Separation of these host-specific proteins while maintaining the biological activity and structural entirety of the desired protein may cause severe problems.

One known approach to overcome the difficulties mentioned is to express the desired protein as a fusion protein, i.e. to the C-terminus or especially N-terminus of the desired protein is bound a stabilizing and/or protective polypeptide. This polypeptide is chosen depending on the problems to be solved (degradation, purification etc.) and the host used for the expression of the fusion protein. Such fusion proteins have proved effective in preventing desired proteins from degradation and in facilitating the purification procedure. There are described numerous polypeptides which have been used as fusion partners in fusion proteins, see for example H.M. Sassenfeld, Trends in Biotechnology 8, 88-93 (1990). Exemplary polypeptides include β-galactosidase, protein A and chloramphenicol acetyltransferase. As in most cases and for obvious reasons (antigenicity etc.) the fusion protein has no practical utility as such it is required to remove the fusion partner from the desired protein after expression and purification (alternatively, the fusion protein can be another heterologous protein and separation leads to two desired proteins). This is generally done by means of a linker sequence linking the desired protein to the fusion partner and containing a cleavage site which can selectively be cleaved by chemical or enzymatic means. Such cleavage sites include, for example, a methionyl radical which is susceptible to the attack of cyanogen bromide, or a polypeptide chain including the tetrapeptidyl radical Asp-Asp-Asp-Lys which is cleaved by enterokinase after Lys. It is obvious that such amino acid subsequences must not occur at the surface of the desired proteins. As the convenient removal of the fusion panner still is the most significant problem to be solved there is a need for alternative means which allow the specific and gentle freeing of the desired protein from the fusion partner. It is an object of the invention to provide such means.

The protease yscF (or KEX2 encoded endoprotease) and the peptidase yscα (or KEX1 encoded carboxypeptidase) have been identified to be responsible for the maturation of the mating α-factor precursor in yeast to yield the mature α-factor pheromone. Protease yscF is a membrane-bound endoprotease which is active in the neutral pH range and is completely dependent on $Ca^{2+}$ ions. It has, near the carboxyterminus, a hydrophobic region which has been identified to be responsible for membrane binding. Removal of this membrane-binding domain yields a soluble ycsF derivative which still retains its enzymatic activity and specificity, viz. cleavage at the C-terminal side of a pair of basic amino acids, such as Lys-Arg or Arg-Arg [cf. R.S. Fuller et al. Proc. Natl. Acad. Sci. USA 86, 1434-1438 (1989). In EP-A-0 252 854, for example, a construct for the production of hirudin is described, comprising the fragment promoter - signal sequence - yscF cleavage site-hirudin. On the other hand, protease yscα is a membrane-bound carboxyexopeptidase which is highly active towards C-terminal basic amino acids (Arg. Lys) at pH between 6 and 7.5. Like yscF, yscα contains a hydrophobic membrane-binding segment in the vicinity of the C-terminus. Removal of this membrane-binding domain leads to a soluble yscα derivative with retained enzymatic activity and specificity [A. Cooper and H. Bussey, Mol. Cell. Biol. 9, 2706-2714 (1989)]. It has now been found that proteases yscF and yscα can advantageously be used in combination for the preparation of mature proteins by in vitro processing of suitably tailored fusion proteins.

Accordingly, the present invention concerns a method for the production of a biologically active protein comprising treating a fusion protein consisting of

1. one or multiple successive protein segment(s) each consisting of said biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and

2. a polypeptide tag joined to the C-terminal amino acid of said successive protein segment(s),

or consisting of

1. a polypeptide tag joined to the N-terminal amino acid of

2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said linker polypeptide sequence L being joined to said biologically active protein,

with soluble yeast endoprotease yscF and with soluble yeast carboxypeptidase yscα, and isolating said biologically active protein.

The fusion protein may be represented by the formula

$$(P\text{-}L)_m\text{-}T \qquad (I)$$

or

$$T\text{-}(L\text{-}P)_n \qquad (II),$$

in which P is the biologically active protein, L is a linker polypeptide sequence as defined above, T is a polypeptide tag, m is an integer from 1 to 10 and n is an integer from 2 to 10.

The biologically active protein may be any protein of biological interest and of prokaryotic or especially eukaryotic, in particular higher eukaryotic such as mammalian (including animal and human), origin and is, for example, an enzyme which can be used, for example, for the production of nutrients and for performing enzymatic reactions in chemistry or molecular biology, or a protein which is useful and valuable for the treatment of human and animal diseases or for the prevention thereof, for example a hormone, polypeptide with immunomodulatory, anti-viral and anti-tumor properties, an antibody, viral antigen, blood clotting factor, a fibrinolytic agent, a growth regulation factor, furthermore a foodstuff and the like.

Example of such proteins are e.g. hormones such as secretin, thymosin, relaxin, calcitonin, luteinizing hormone, parathyroid hormone, adrenocorticotropin, melanocyte-stimulating hormone, β-lipotropin, urogastrone, insulin, growth factors, such as epidermal growth factor (EGF), insulin-like growth factor (IGF), e.g. IGF-I and IGF-II, mast cell growth factor, nerve growth factor, glia derived nerve cell growth factor, platelet derived growth factor (PDGF), or transforming growth factor (TGF), such as TGFβ, growth hormones, such as human or bovine growth hormones, interleukin, such as interleukin-1 or -2, human macrophage migration inhibitory factor (MIF), interferons, such as human α-interferon, for example interferon-αA, αB, αD or αF, β-interferon, γ-interferon or a hybrid interferon, for example an αA-αD- or an αB-αD-hybrid interferon, especially the hybrid interferon BDBB, proteinase inhibitors such as $\alpha_1$-antitrypsin, SLPI and the like, hepatitis virus antigens, such as hepatitis B virus surface or core antigen or hepatitis A virus antigen, or hepatitis nonA-nonB antigen, plasminogen activators, such as tissue plasminogen activator or urokinase, hybrid plasminogen activators, such as $K_2$tuPA, tick anticoagulant peptide (TAP), tumour necrosis factor, somatostatin, renin, immunoglobulins, such as the light and/or heavy chains of immunoglobulin D, E or G, or human-mouse hybrid immunoglobulins, immunoglobulin binding factors, such as immunoglobulin E binding factor, human calcitonin-related peptide, blood clotting factors, such as factor IX or VIIIc, platelet factor 4, erythropoietin, eglin, such as eglin C, desulfatohirudin, such as desulfatohirudin variant HV1, HV2 or PA, corticostatin, echistatin, cystatins, human superoxide dismutase, viral thymidin kinase, β-lactamase or glucose isomerase. Preferred genes are those coding for a human α-interferon e.g. interferon αB, or hybrid interferon, particularly hybrid interferon BDBB (see EP 205,404), human tissue plasminogen activator (t-PA), human single chain urokinase-type plasminogen activator (scu-PA), hybrid plasminogen activator $K_2$tuPA (see EP 277,313), transforming growth factor β, human calcitonin, insulin-like growth factor I and II and desulfatohirudin, e.g. variant HV1. Proteins containing a pair of basic amino acids, such as Arg-Arg, Lys-Arg, Lys-Lys and Arg-Lys, exposed on the protein surface and therefore amenable to proteolytic cleavage, are not suited for the process according to the invention and will have to be mutated such that one of the consecutive basic amino acids is replaced by another non-basic amino acid without affecting the biological activity. Proteins having a C-terminal basic amino acid which is essential for biological activity cannot be produced by the process according to the invention (because this basic amino acid is removed by soluble yscα) unless an additional non-basic protective amino acid is added to the C-terminus of such proteins.

The linker polypeptide sequence L comprises 2 to about 20, especially 2 to 12, amino acid residues and contains one or multiple, especially 1 to 5, pairs of basic amino acids, such as Arg-Arg, Lys-Arg, Lys-Lys or Arg-Lys, provided that in compounds of the formula I (m >1) and II the N-terminal first and second amino acids as well as the C-terminal penultimate and ultimate amino acids represent such a pair of basic amino acids while in compounds of the formula I (m = 1) it is sufficient that solely the N-terminal first and second amino acids represent such a pair of basic amino acids. The choice of the amino acid residues linking the individual pairs of basic amino acids and/or joining the pair(s) of basic amino acid residues to the polypeptide tag (cf. compounds of the formula I with m =1 ) is not crucial. For example, any of the genetically encoded neutral amino acids may be chosen for that purpose. The most simple linker polypeptide sequence L is a dipeptidyl radical selected from Arg-Arg, Lys-Arg, Lys-Lys and Arg-Lys.

Since the extension of a small or medium-sized biologically active protein has proved to have a favourable influence on stability the polypeptide tag T may, in principle, be any synthetic polypeptide imaginable or any naturally occurring polypeptide or part thereof. Preferably, the polypeptide tag T consists of about 10 to about 1000, in particular 30 to 300, amino acid residues and represents a full-length polypeptide which is well-expressed in the host used for expression of the fusion protein (see below) or a part thereof. If it is the main object to facilitate the purification it is preferable to use a polypeptide tag which is susceptible for affinity chromatography (the polypeptide tag is, for example, recognized by an available monoclonal or polyclonal antibody or is bound by a specific material such as cellulose) or ion exchange chromatography (the polypeptide tag comprises a large number of acidic or basic amino acid residues). Examples of such polypeptide tags include, for example, yeast acid phosphatase PH05, yeast invertase or yeast carboxypeptidase Y especially when yeast is used as the host, polymerase of MS2 phage, β-galactosidase or E. coli acid phosphatase especially when E. coli is used as the host, neomycin, phosphotransferase, dehydrofolate reductase, an immunoglobulin or a lectin especially when mammalian host cells are used, furthermore desulfatohirudin, eglin C, chymosin, interleukin I, the Exg protein of Cellulomonas fimi, and the like, it also being possible to use fragments of these polypeptides.

Preferably, m is an integer from 1 to 5 and n is an integer from 2 to 5.

Soluble yeast endoprotease yscF and yeast carboxypeptidase yscα are muteins of yscF and yscα in which the hyrophobic membrane binding sites have been deleted. The amino acid sequence of the 814-residue protease yscF is known from K. Mizuno et al. [Biochem. Biophys. Res. Commun. 156, 246-254 (1988)]. The membrane binding site is located in the region $Tyr^{679}$ to $Met^{699}$. In the soluble yscF endoproteases according to the invention the membrane binding site has selectively been removed hence the C-terminus starting with, for example, amino acid 700 (Lys) is still present, or the whole C-terminus including the membrane binding site, i.e. 136 to approximately 200 amino acids from the C-terminus, has been removed. Such soluble yscF deletion muteins are described, for example, in EP 327,377 or in R.S. Fuller et al. (supra). The amino acid sequence of the 729-residue peptidase yscα is likewise known [A. Dmochowska et al., Cell 50, 573-584 (1987)]. The membrane binding site is located in the region $Ala^{619}$ to $Tyr^{637}$. As above, in the soluble yscα carboxypeptidases according to the invention the membrane binding site has selectively been removed hence leaving the C-terminus starting with, for example, amino acid 638 (Asp) intact, or the whole C-terminus including the membrane binding site, i.e. 93 to approximately 110 amino acids from the C-terminus, has been removed. One such soluble yscα carboxypeptidase mutein has been described by A. Cooper and H. Bussey (supra). The preferred soluble yscF carboxypeptidase according to the invention has the sequence depicted in the sequence listing under SEQ ID No. 1 while the preferred soluble yscα carboxypeptidase has the sequence depicted in the sequence listing under SEQ ID No. 2.

The digestion of the fusion proteins with soluble yscF and soluble yscα is performed using conditions under which yscF and yscα are known to work best, i.e. in a buffered solution at pH from about 6.0 to about 7.5, preferably at about 7.0, in a temperature range of from about 25°C to about 37°C and for about 1 to 4 hours, preferably until the digestion is complete as judged by HPLC control. As yscF is strongly dependent on $Ca^{2+}$ ions, a calcium salt, such as calcium chloride, is added to the digestion mixture. The molar ratios of fusion protein : soluble yscF : soluble yscα is in the range 1:1:1 to $10^4$:1:1. The molar concentration of $Ca^{2+}$ ions is in the range of from 0.1 mM to 10 mM. Optionally, a low concentration (< 1 %) of an non-ionic detergent, such as Triton X-100, may be added to the mixture as yscF is known to be activated thereby. The fusion protein may be treated with a digestive mixture containing both soluble yscF and soluble yscα, or the fusion protein may first be treated with soluble yscF and, when the digestion has sufficiently proceeded or is complete, thereupon with soluble yscα, preferably in situ, i.e. without isolating the product of the first digestion step.

The biologically active protein can be isolated from the digestion mixture using conventional means. Suitable purification steps include, for example, desalination, chromatographic processes such as ion exchange chromatography, gel filtration chromatography, partition chromatography, HPLC, reversed phase HPLC, gel electrophoresis, carrier-free electrophoresis, affinity chromatography such as affinity chromatography with monoclonal antibodies coupled to an insoluble matrix, and the like, or any combination thereof.

In case the desired protein does not assume the correct three-dimensional structure due, for example, to an incorrect formation of disulfide bonds (if cysteine residues are present), it may be necessary to solubilize it under conditions which are suitable in maintaining it in its denatured form and subsequently to refold it with the concomitant coupling of disulfide bonds (if cysteine residues are present). Appropriate methods are known for a large number of proteins. Otherwise, it is required to adapt methods known in the art to the specific problems encountered.

As mentioned above some soluble yscF and yscα deletion muteins are known from the literature. Further deletion muteins according to the invention can be prepared using methods known in the art, for example by preparing a corresponding DNA coding for said mutein, inserting it in a suitable vector DNA under the control of an expression control sequence, transforming suitable host microorganisms with the expression vector formed, culturing the transformed host microorganism in a suitable culture medium and isolating the produced mutein. The DNA coding for any of said muteins can be produced for example, by taking a plasmid containing the DNA coding for yscF (KEX2) or yscα (KEX1 ) and 1. digesting it with a restriction enzyme which cleaves within or 3′ of the DNA region coding for the membrane binding site (for example, EcoRI, BstXI or NarI in the case of KEX2 and HgiAI, TaqII or ClaI in the case of KEX1), digesting the cleaved DNA with a suitable endonuclease, for example Bal31, such that said DNA region is removed and recircularizing the linearized plasmid by blunt end ligation or the like, or 2. choosing or creating (for example by site-directed mutagenesis) one restriction site 5′ to and one restriction site 3′ to the DNA region coding for the membrane binding site (for example PvuII and NarI or EcoRI n the case of KEX2, XhoI, StuI or XcaI in the case of KEX1; the 3′restriction site may also be located within the plasmid DNA adjacent to the translation stop signal of the KEX2 or KEX1 gene), digesting the plasmid with two restriction enzymes recognizing said restricting sites and recircularizing the linearized plasmid by blunt end ligation or the like, or 3. deleting the DNA region coding for the membrane binding site by using loop-out mutagenesis, or 4. totally deleting the C-terminus by digesting with PvuII in the case of KEX2 and with XhoI, StuI or ScII in the case of KEX1, respectively, and recircularizing the linearized plasmid by blunt end ligation or the like. As the DNA sequences of KEX2 and KEX1 are known (K. Mizumo et al., A. Dmochowska et al., supra) a suitable mutagenic oligonucleotide can easily be devised and used to delete said DNA region applying the M13 cloning system. Care must be taken that the mutated KEX2 or KEX1 genes include a translation stop signal (TAA, TAG or TGA). If necessary, such a stop signal can be introduced at the desired place via a synthetic linker DNA or it may be provided by the adjacent vector DNA. Accordingly, the mutated KEX2 and KEX1 genes may include at their 3′ ends codons derived from the vector DNA which code for a few (such as 2 to 20) additional amino acids at the C-termini of the soluble yscF and yscα muteins. All of these methods make use of conventional techniques.

<u>Preparation of the fusion protein</u>

The fusion proteins according to the invention can be prepared by recombinant DNA technique comprising culturing a transformed host under conditions which allow expression thereof and isolating the fusion protein. More specifically, the desired compounds are manufactured by

a) providing an expression vector comprising an expression cassette containing a DNA sequence coding for said fusion protein,

b) transferring the expression vector into a recipient host,

c) culturing the transformed host under conditions which allow expression of the fusion protein, and

d) isolating the fusion protein.

The steps involved in the preparation of the fusion proteins by recombinant DNA technique will be discussed in more detail hereinbelow.

<u>Expression vectors</u>

The invention relates to expression vectors comprising an expression cassette containing a DNA sequence coding for a fusion protein consisting of 1. one or multiple successive protein segment(s) each consisting of a biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and 2. a polypeptide tag joined to the C-terminal amino acid of said successive protein segment(s), or consisting of 1. a polypeptide tag joined to the N-terminal amino acid of 2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said linker polypeptide sequence L being joined to said biologically active protein, and a method for the preparation thereof.

The expression cassette may be represented by the formula

$$Pr\text{-}S\text{-}(D_P\text{-}D_L\text{-}D_T)_m\text{-}T \qquad (III)$$

wherein Pr is the expression control sequence, S is a bond or represents a DNA sequence encoding a signal peptide, $D_P$ represents a DNA sequence coding for a biologically active protein, $D_L$ is a DNA sequence coding for the linker polypeptide L wherein the first and second codons adjacent to the 3' end of $D_P$ and, if m is different from 1, also the penultimate and ultimate codons adjacent to the 5' end of $D_T$ code for basic amino acids selected from Lys and Arg, $D_T$ represents a DNA sequence coding for a polypeptide tag and T represents a DNA sequence containing transcription termination signals, wherein S, $D_P$, $D_L$ and $D_T$ are in the same reading frame, and m is an integer from 1 to 10, or by the formula

$$Pr\text{-}S\text{-}(D_T\text{-}D_L\text{-}D_P)_n\text{-}T \qquad (IV)$$

wherein Pr, S, $D_T$, $D_L$, $D_P$ and T have the meanings given above, and wherein S, $D_T$, $D_L$ and $D_P$ are in the same reading frame, and n is an integer from 2 to 10.

Constructs in which S represents a DNA sequence encoding a signal peptide are preferred.

The vector is selected depending on the host cells envisaged for transformation. In principle, all vectors which replicate and express the gene according to the invention in the chosen host are suitable. Examples of suitable hosts are prokaryotes and eukaryotes, which are devoid of or poor in restriction enzymes or modification enzymes and which are devoid of yscF or yscF-related activity, such as bacteria, for example Escherichia coli or Bacillus subtilis, yeasts, for example Saccharomyces cerevisiae, especially kex2 mutants thereof, and furthermore mammalian cells, in particular established human or animal cell lines, e.g. myeloma cells, human embryonic lung fibroblasts L-132, mice LTK cells, human malignant melanoma Bowes cells, HeLa cells, SV-40 virus transformed kidney cells of African green monkey COS-7 or chinese hamster ovary (CHO) cells and variants thereof. Chinese hamster orary cells and strains of Escherichia coli and Saccharomyces cerevisiae are preferred as the host microorganism.

Vectors for use in yeast and E. coli.

Examples of vectors that are suitable for the expression of the fusion protein gene in an E. coli strain are bacteriophages, for example derivatives of the bacteriophage λ, or plasmids, such as the plasmid co1E1 and its derivatives, for example pMB9, pSF2124, pBR317 or pBR322. Suitable vectors contain a complete replicon and a marker gene, which renders possible the selection and identification of the microorganisms transformed by the expression plasmids by means of a phenotype feature. Suitable marker genes impart to the microorganism, for example, resistance to heavy metals, antibiotics such as ampicillin or tetracyclin, and the like.

Several expression control sequences Pr can be used for regulating the expression cassette in E. coli. Especially promoters of strongly expressed genes are used. Suitable promoters are the lac, tac, trp and lpp promoters, furthermore the phage λN or the phage λpL promoter, and others. In the present invention, the preferred promoter for use in E. coli is the λPL, trp and the lac promoter.

Vectors suitable for replication and expression in S. cerevisiae contain a yeast-replication origin and a selective genetic marker for yeast. Hybrid vectors that contain a yeast replication origin, for example the chromosomal autonomously replicating segment (ARS), are retained extrachromosomally within the yeast cell after transformation and are replicated autonomously during mitosis. Also, hybrid vectors that contain sequences homologous to the yeast 2μ plasmid DNA or that contain ARS and a sequence of a chromosomal centromer, for example CEN4, can be used. Suitable marker genes for yeast are especially those that impart antibiotic resistance to the host or, in the case of auxotrophic yeast mutants, genes that complement the host lesions. Corresponding genes impart, for example, resistance to the antibiotic cycloheximide or provide for prototrophy in an auxotrophic yeast mutant, for example the URA3, LEU2, HIS3 or the TRPI gene.

Preferably, yeast hybrid vectors furthermore contain a replication origin and a marker gene for a bacterial host, especially E. coli, so that the construction and the cloning of the hybrid vectors and their precursors can be carried out in E. coli. Expression control sequences Pr suitable for expression in yeast are, for example, those of the CYC1, GAL1/10 or PH05 gene, and also promoters involved in glycolysis, for example the PGK or the GAP (including 5′ truncated GAP) promoter, furthermore the α-factor promoter and hybrid promoters, such as hybrid PH05-GAP promoters.

The DNA sequence encoding a signal peptide S ("signal sequence") is derived from a gene of the microbial host coding for a polypeptide which is ordinarily secreted. When E. coli is used as the host microorganism the ompA, lpp, maltose binding protein, λ receptor, acid phosphatase or β-lactamase signal sequence may be chosen. Suitable signal sequences S for use in yeast are, for example, the signal and prepro sequences of the yeast invertase, pheromone peptidase (KEX1 ), "killer toxin" and repressible acid phosphatase (PH05) genes, the α-factor leader and the glucoamylase signal sequence from Aspergillus awamori. Alternatively, fused signal sequences may be constructed by ligating part of the signal sequence (if present) of the gene naturally linked to the promoter used (for example PH05), with part of the signal sequence of the biologically active protein (if present). Those combinations are favoured which allow a precise cleavage between the signal peptide and the fusion protein amino acid sequence.

The DNA sequence containing transcription termination signals T is preferably the 3′ flanking sequence of a gene derived from the selected microbial host which contains proper signals for transcription termination. Suitable 3′ flanking sequences are, for example, those of the gene naturally linked to the promoter used.

Vectors for use in mammalian cells

Vectors for replication and expression in mammalian cells are frequently provided with DNA from viral origin, e.g. from simian virus 40 (SV 40), Rous sarcoma virus (RSV), adenovirus 2, bovine papilloma virus (BPV), papovavirus BK mutant (BKV), or mouse or human cytomegalovirus (MCMV and HCMV, respectively).

Expression control sequences Pr which are suitable for use in mammalian cells include, inter alia, the early and late promoters of SV40, the major late promoter of adenovirus, the promoter of the murine metallothionein gene and the enhancer-promoter region of the mouse or human cytomegalovirus major immediate-early gene, the human immunoglobulin enhancer-promoter region, the human $\alpha$-globin promoter optionally combined with the SV40 enhancer and promoters derived from the heat shock genes. Signal sequences S for use in mammalian cells are, for example, the signal sequences derived from influenza haemagglutinin, human t-PA and prepro-insulin.

Suitable marker genes for mammalian cells are, for example, the neo and ble genes from transposon Tn5 which confer resistance to the antibiotic G418 and to bleomycin-type antibiotics, respectively, the E.coli gene for hygromycin-B resistance, the dihydrofolate reductase gene (dhfr) from mammalian cells or E.coli which changes the phenotype of $DHFR^-$ cells into $DHFR^+$ cells and/or confer resistance to methotrexate, and the thymidine kinase gene of herpes simplex virus which makes $TK^-$ cells phenotypically $TK^+$ cells.

Preferably, the hybrid vectors for mammalian cells contain the 3′ untranslated region of a mammalian gene containing signals for proper transcription termination and polyadenylation (T), such as, for example, the 3′flanking region of the $\beta$-globin gene. Advantageously, the regions flanking the polypeptide coding region include one or more native introns having the appropriate splicing signals at their termini. Such additions are deemed necessary as cDNAs and prokaryotic DNAs such as the above selection genes, generally lack such transcription and processing signals.

Preferably, such vectors contain an origin of replication and an antibiotic resistance gene for propagation in E.coli. A mammalian origin of replication may be provided either by including in the construction of the vector a eukaryotic origin, such as derived from SV40 or from another viral source, or may be provided by the host cell chromosome upon integration of the vector into the host cell chromosome.

The DNA sequences $D_P$ and $D_T$ coding for the biologically active protein and the polypeptide tag are known or, if not, can be deduced from known amino acid sequences and produced synthetically applying methods known per se. The DNA sequence $D_L$ coding for the linker polypeptide L is preferably a synthetic DNA sequence containing pair(s) of basic amino acids as specified above and is prepared synthetically using conventional synthetic methods.

In the expression cassette according to the invention the coding sequences S, $D_P$, $D_L$ and $D_T$ are joined together in one uninterrupted reading frame starting with an ATG at the 5′ end and terminating with a translation stop signal (TAA,TAG or TGA) at the 3′ end. The coding sequences $S\text{-}(D_P\text{-}D_L\text{-}D_T)_m$ and $S\text{-}(D_T\text{-}D_L\text{-}D_P)_n$ are operably linked to the expression control sequence Pr.

The expression vectors according to the invention are prepared by methods known in the art applying conventional ligation techniques, for example by linking the expression cassette (prepared previously) as such or the components of the expression cassette successively in the predetermined order to the vector DNA. The components of the expression vectors are linked through common restriction sites and/or by means of synthetic linker molecules and/or by blunt end ligation.

Transformed hosts

Another aspect of the present invention involves host cells transformed with an expression vector comprising an expression cassette containing a DNA sequence coding for a fusion protein consisting of 1. one or multiple successive protein segment(s) each consisting of a biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and 2. a polypeptide tag joined to the C-terminal amino acid of said successive protein segment(s), or consisting of 1. a polypeptide tag joined to the N-terminal amino acid of 2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said tinker polypeptide sequence L being joined to said biologically active protein, and a method for the preparation thereof.

Examples of suitable hosts including prokaryotic and eukaryotic hosts are those specified above. The method for the preparation of transformed host cells comprises transforming host cells with the above expression vector.

The transformation of the eukaryotic host cells is accomplished by methods known in the art. For example, the transformation of yeast with the hybrid vectors may be accomplished according to the method described by Hinnen et al [Proc. Natl. Acad. Sci. USA 75, 1919(1978)]. This method can be divided into three steps:

(1) Removal of the yeast cell wall or parts thereof.
(2) Treatment of the "naked" yeast cells (spheroplasts) with the expression vector in the presence of PEG (poly-

ethyleneglycol) and $Ca^{2+}$ ions.

(3) Regeneration of the cell wall and selection of the transformed cells in a solid layer of agar.

The introduction of expression vectors into mammalian cells is done by transfection in the presence of helper compounds, e.g. diethylaminoethyldextran, dimethyl sulfoxide, glycerol, polyethylene glycol or the like, or as co-precipitates of vector DNA and calcium phosphate. Further suitable methods include direct microinjection of vector DNA into the cell nucleus and electroporation, i.e. introduction of DNA by a short electric pulse increasing the permeability of cell membranes. The subsequent selection of transfected cells can be done using a selection marker which is either covalently integrated into the expression vector or added as a separate entity. The selection markers include genes which confer resistance to antibiotics or genes which complement a genetic lesion of the host cell (supra).

The transformation of the bacterial host strains with the expression vectors according to the invention is carried out, for example, in the manner described in the literature for B. subtilis [Anagnostopoulos et al., J. Bacteriol. 81, 741 (1961)] and E.coli [M. Mandel et al., J. Mol. Biol. 53, 159 (1970)]. The isolation of the transformed host cells is effected advantageously from a selective nutrient medium to which there has been added, for example, the biocide against which the marker gene contained in the expression plasmid imparts resistance. If, for example, the hybrid vectors contain the $amp^R$ gene, ampicillin is accordingly added to the nutrient medium. Cells that do not contain the hybrid vector are destroyed in such a medium.

Cultivation of transformed host cells

The invention concerns furthermore a method for the production of a fusion protein consisting of 1. one or multiple successive protein segment(s) each consisting of a biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and 2. a polypeptide tag joined to the C-terminal amino acid of said successive protein segment(s) or consisting of 1. a polypeptide tag joined to the N-terminal amino acid of 2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said tinker polypeptide sequence L being joined to said biologically active protein, comprising culturing under appropriate nutrient conditions a transformed host cells containing an expression vector comprising a DNA sequence coding for said fusion protein and isolating said fusion protein.

The transformed host cells are cultured by methods known in the art in a liquid medium containing assimilable sources of carbon, nitrogen and inorganic salts. Various sources of carbon can be used for culture of the transformed E. coli and yeast cells according to the invention. Examples of preferred sources of carbon are assimilable carbohydrates, such as glucose, maltose, mannitol or lactose, or an acetate, which can be used either by itself or in suitable mixtures. Examples of suitable sources of nitrogen are amino acids, such as casaminoacids, peptides and proteins and their degradation products, such as tryptone, peptone or meat extracts, yeast extracts, malt extract and also ammonium salts, for example ammonium chloride, sulfate or nitrate, which can be used either by themselves or in suitable mixtures. Inorganic salts which can also be used are, for exemple, sulfates, chlorides, phosphates and carbonates of sodium, potassium, magnesium and calcium.The medium furthermore contains, for exemple, growth-promoting substances, such as trace elements, for example iron, zinc, manganese and the like, and preferably substances which exert a selection pressure and prevent the growth of cells which have lost the expression plasmid. Thus, for example, if a yeast strain which is auxotrophic in, for example, an essential amino acid, is used as the host microorganism, the plasmid preferably contains a gene coding for an enzyme which complements the host defect. Cultivation of the yeast strain is performed in a minimal medium deficient in said amino acid.

Culturing is effected by processes which are known in the art. The culture conditions, such as temperature, pH value of the medium and fermentation time, are chosen such that a maximum titre of the fusion proteins of the invention is obtained. Thus, the yeast strain is preferably cultured under aerobic conditions by submerged culture with shaking or stirring at a temperature of about 20 to 40°C, preferably about 30°C, and a pH value of 5 to 8, preferably at about pH 7, for about 4 to 30 hours, preferably until maximum yields of the proteins of the invention are reached.Mammalian cells are grown under tissue culture conditions using commercially available media optionally supplemented with growth-promoting substances and/or mammal sera. The cells are grown either attached to a solid support, e.g. a microcarrier or porous glass fibres, or free-floating in appropriate culture vessels. The culture medium is selected in such a way that selection pressure is exerted and only those cells survive which still contain the hybrid vector DNA including the genetic marker. Thus, for example, an antibiotic is added to the medium when the hybrid vector includes the corresponding antibiotic resistance gene.

When the cell density has reached a sufficient value culturing is interrupted and the protein isolated. If the expression cassette comprises a signal sequence the desired fusion protein may be secreted into the medium. The medium containing the product is separated from the cells which can be provided with fresh medium and used for continuous production. When yeast cells or E. coli cells are used the protein can also accumulate within the cells, especially in the periplasmic space. In the latter case the first step for the recovery of the fusion protein consists in liberating the protein from the cell interior. The cell wall is first removed by enzymatic digestion with glucosidases (supra) or, alternatively, the cell wall is removed by treatment with chemical agents, i.e. thiol reagents or EDTA, which give rise to cell wall damages permitting the produced protein to be released. The resulting mixture is enriched for fusion protein by conventional means, such as removal of most of the non-proteinaceous material by treatment with poly-ethyleneimine, precipitation of the proteins using ammonium sulphate, gel electrophoresis, dialysis, chromatography, for example, ion exchange chromatography (especially preferred when the polypeptide tag of the fusion protein includes a large number of acidic or basic amino acids), size-exclusion chromatography, HPLC or reverse phase HPLC, molecular sizing on a suitable Sephadex® column, or the like. The final purification of the pre-purified product is achieved, for example, by means of affinity chromatography, for example antibody affinity chromatography, especially monoclonal antibody affinity chromatography using antibodies fixed on an insoluble matrix by methods known in the art, which antibodies recognize, for example, epitopes located within the polypeptide tag of the fusion protein.

The invention concerns furthermore the fusion proteins according to the invention per se which are valuable intermediates for the production of biologically active proteins.

The invention concerns especially the expression vectors, the transformed hosts, the fusion proteins and the methods for the preparation thereof and the method for the preparation of a biologically active protein as described in the examples.

The following examples serve to illustrate the invention but should not be construed as a limitation thereof.

Example 1: Construction of a shortened KEX2 gene encoding soluble yscF variant

The yeast KEX2 gene codes for an endoprotease called yscF, which is a membrane bound protein localized in the Golgi apparatus. The yscF protein consists of a N-terminal catalytic domain, a Ser/Thr rich domain, a membrane spanning domain and a C-terminal tail responsible for Golgi localization of the yscF protein. Mutant yscF enzyme lacking 200 C-terminal amino acids, including the Ser/Thr rich domain, the membrane spanning domain and the C-terminal tail still retains protease activity [Fuller et al., 1989, Proc. Natl. Acad. Sci. 86, 1434-1438; Fuller et al., 1989, Science 246, 482-485]. In order to get a soluble yscF protease activity, a mutant KEX2 gene lacking 600 bp, coding for the C terminal 200 amino acids, is constructed. The truncated gene is under the control of the KEX2 promoter reaching from -1 to -502. Translation is terminated at a stop codon (TAA) originating from the polylinker of pUC18.

In detail, plasmid pUC19 [Boehringer Mannheim GmbH, FRG] is digested to completion with HindIII and the 2686 bp fragment is isolated. The ends are filled in and the fragment is religated. An aliquot of the ligation mixture is added to calcium-treated, transformation competent E.coli JM101 [Invitrogen, San Diego, USA] cells. 12 transformed ampicillin resistant E.coli transformants are grown in the presence of 100 µg/ml ampicillin. Plasmid DNA is prepared and analysed by digestion with HindIII as well as with BamHI. The plasmid lacking the HindIII site is designated pUC19woH.

A 3207 bp BalI-AhaIII KEX2 fragment (obtainable from total genomic yeast DNA) is provided at both ends with BamHI linkers followed by a complete digestion with BamHI. Plasmid pUC19woH is cut to completion with BamHI, the linear 2690 bp fragment is isolated and ligated to the BamHI KEX2 fragment described above. An aliquot of the ligation mixture is transformed into E.coli JM101 cells. 12 transformed, ampicillin resistant colonies are grown in ampicillin (100 µg/ml) containing LB medium, plasmid DNA is extracted and analyzed by BamHI digests. One clone with the expected restriction fragments is selected and called pKS301 b.

The 2 µm yeast vector pAB24 which corresponds essentially to plasmid pDP34 is cut to completion with BamHI and the linear pAB24 fragment is isolated. Plasmid pKS301b is digested with BamHI and the fragment containing the complete KEX2 gene is isolated and ligated to the linearized yeast vector pAB24. An aliquot of the ligation mixture is transformed into E.coli JM101 and plasmid DNA of twelve positive clones is examined by BamHI digests. One clone with the expected restriction fragments is referred to as pAB226.

Plasmid pKS301b is digested to completion with SphI, PvuII and ScaI. The 2.37 kb SphI-PvuII fragment containing KEX2 sequences from -502 to +1843 and a part of the pUC19 polylinker is isolated. Plasmid pUC18 [Boehringer Mannheim, FRG] is cut to completion with SphI and SmaI. The 2660 bp SphI-SmaI pUC18 fragment is ligated to the 2.37 kb SphI-PvuII KEX2 fragment by SphI/SphI and PvuII/SmaI ligation. The PvuII/SmaI ligation results in the fusion of the KEX2 ORF coding for 614 amino acids to an ORF in the pUC18 sequences which codes for 7 additional C-terminal amino acids (-G-V-P-S-S-N-S) and is followed by a stop codon (TAA). An aliquot of the ligation mixture is transformed into E.coli JM101. Plasmid DNA is isolated from ampicillin resistant E.coli transformants and analyzed by digestion with SphI and EcoRI as well as with HindIII. One clone with the expected restriction pattern is referred to as p18kexp. In the sequence listing under SEQ ID No. I the ORF encoding soluble yscF with KEX2-derived DNA is shown.

Plasmid p18kexp is cut to completion with PvuII, SalI and ScaI. The 2552 bp SalI-PvuII fragment containing the KEX2 sequences reaching from -502 to +1843 as well as 206 bp of pUC18 sequences is isolated. Plasmid pDP34 is digested with BamHI and the ends of the linearized plasmid are filled in. After inactivation of T4 polymerase the linearized filled-in plasmid is cut with SalI and the 11.78 kb fragment is isolated. The pDP34 BamHI*-SalI fragment is ligated to the 2552 bp SalI-PvuII fragment by SalI/SalI and BamHI*/PvuII ligation (BamHI*: filled-in BamHI). An aliquot of the ligation mixture is transformed into transformation competent E.coli JM101 cells. Plasmid DNA is extracted from ampicillin resistant cells and analyzed by restriction analysis with SalI, NcoI, SmaI, XbaI, EcoRI. One clone with the expected restriction fragments is referred to as pDPkexp.

Example 2: Assay of endoprotease activity

Cells are cultured as described in Example 3. The culture broth is centrifuged and separated in cells and culture medium. Endoprotease activity is determined in the culture medium, on the surface of intact cells and in "permeabilized" cells. The cells are washed once with one volume 0.2 M HEPES (4-(2-Hydroxyethyl)-piperazine-1-ethane sulfonic acid, Fluka, Switzerland) pH 7.0 and resuspended in one volume 0.2 M HEPES pH 7.0 for measurements on intact cells or resuspended in 0.2 M HEPES pH 7.0 containing 0.1% Triton X-100 in order to produce "permeabilized" cells. Endoprotease activity is measured as follows: 900 μl of assaymix (0.2 M HEPES pH 7.0, 1 mM $CaCl_2$, 0.5 mM PMSF [for measuring activity of permeabilized cells additional 0.1% Triton X-100]) is incubated with 50 μl sample at 37°C for 2 minutes and $A_{405nm}$ increase during this incubation period is spectrophotometrically determined. No change in $A_{405}$ can be observed during this preincubation without substrate. The reaction is started by adding the substrate (50 μl of 10 mM benzyloxycarbonyl-L-tyrosyl-L-lysyl-L-arginine-4-nitroanilide, Bachem, Bubendorf, Switzerland) and the increase in $A_{405}$ is taken for calculation of endoprotease activity. 1U endoprotease activity is defined as 1 μmol 4-nitroanilide produced per minute under the described assay conditions. About 90% of yscF activity is found in the culture medium.

Example 3: Transformation of S.cerevisiae strain AB110

Saccharomyces cerevisiae strain AB110 (ATCC 20796) is transformed with plasmids pDP34, pAB226 and pDPkexp using the transformation protocol described by Dohmen et al. [1989, Curr. Genet. 15, 319-325]. Transformed yeast cells are selected on yeast minimal plates supplemented with an amino acid/nucleotide mix deficient in uracil (amino acid/nucleotide mix supplements the culture medium with adenine 12 mg/l, arginine 40 mg/l, histidine 40 mg/l, isoleucine 60 mg/l, leucine 60 mg/l, lysine 60 mg/l, methionine 40 mg/l, phenylalanine 50 mg/l, threonine 60 mg/1, tryptophan 40 mg/l, tyrosine 15 mg/l, uracil 40 mg/l, valine 60 mg/l). Single transformed yeast clones are isolated and referred to as:

Saccharomyces cerevisiae AB110/pDP34
Saccharomyces cerevisiae AB110/pAB226
Saccharomyces cerevisiae AB110/pDPkexp

Example 4: Fermentation of transformed yeast strains on a laboratory scale

Cells of S. cerevisiae AB110/pDP34, S. cerevisiae AB110/pAB226 and S. cerevisiae AB110/pDPkexp are grown in a 10 ml preculture composed of

| | |
|---|---|
| Difco Yeast Nitrogen Base w/o Amino Acids | 6.7 g/l |
| glucose | 20.0 g/l |
| HEPES | 24.0 g/l. |

The culture medium is supplemented with an amino acid/nucleotide mix deficient in leucine (described in Example 3) and the pH is set to 7.0.

The precultures are grown for 48 h at 30°C and 180 r.p.m..

The main culture medium is composed of:

| | |
|---|---|
| Difco Yeast Nitrogen Base w/o Amino Acids | 6.7 g/l |
| glucose | 40.0 g/l |
| arginine | 8.0 g/l |
| Difco Casaminoacids | 8.5 g/l |

(continued)

| | |
|---|---|
| HEPES<br>pH is set to 7.0. | 24.0 g/l, |

The main cultures are inoculated with about 1% v/v of the precultures and incubated at 30°C and 180 r.p.m.. At several time points during the fermentation aliquots of the cultures are taken and analyzed for cell density, pH of the culture broth and endoprotease activity. Figure 1 shows the intracellular and extracellular yscF activities synthesized by the S.cerevisiae AB110/pDPkexp transformant proving that a soluble secreted yscF variant is expressed in comparison to the transformant AB110/pDP34 which harbors only the yeast vector and the transformant AB110/pAB226 which expresses the entire yscF protein.

Example 5: Immunological demonstration of a truncated soluble yscF protein in the culture medium of S.cerevisiae AB110/pDPkexp transformants

Strains S.cerevisiae AB110/pDPkexp and AB110/pDP34 are grown as described in example 4. The culture broths are separated in cells and culture supernatants by centrifugation. The culture supernatants are concentrated using Centricon 30000 filters (Amicon) to 1/5 of the original volume. Different amounts of the concentrated supernatants are reduced with 1,4-dithio-DL-threitol and separated on a 8% polyacrylamide gel under denaturing conditions. The proteins are either stained with Coomassie brilliant blue or blotted onto a nitrocellulose filter (Western blot). The methods for PAGE and Western blot are described in Sambrook et al., 1989, Molecular Cloning, 2nd edition, Cold Spring Harbor Laboratory Press, New York.

The nitrocellulose filter is incubated at 37°C for 2h in 10 mM $NaH_2PO_4$, 150 mM NaCl, 1% BSA, pH7.2 followed by a second incubation under the same conditions in the same solution which now contains additional polyclonal antibodies raised in rabbits against a lacZ-yscF fusion. (Production and purification of antibodies is described in Sambrock et al., 1989, Molecular Cloning, 2$^{nd}$ edition, Cold Spring Harbor Laboratory Press, New York.) The filter is washed three times for 30 minutes at 37°C in 10 mM $NaH_2PO_4$, 150 mM NaCl, 1% Triton X-100, 0.1% BSA, pH 7.2. These washings are followed by a hybridization reaction with Goat anti-rabbit Ig's alkaline phosphatase (TAGO, Inc., Burlingame, CA), an alkaline phosphatase which is coupled to an antibody specific for the constant regions of antibodies raised in rabbits. This reaction is performed in 10 mM $NaH_2PO_4$, 150 mM NaCl, 0.01% Tween 20, 0.1% BSA, pH 7.2, containing the anti-rabbit Ig's alkaline phosphatase. The blot is washed three times for 30 minutes in 10 mM $NaH_2PO_4$, 150 mM NaCl, 0.01% Tween 20, 0.1% BSA, pH 7.2. Visualization of the specific binding occurs by incubation of the blot in a solution composed of: 0.1 M Tris-HCl pH8.8, 100 mM NaCl, 2mM $MgCl_2$, 10 mg/100 ml 5-Brom-4-Chlorindolyl-Phosphat (BCIP) and 100 mg/100ml Nitroblue-Tetrazolium-Chloride (NBT). An additional 68 kDa protein is found in the culture supernatants of S.cerevisiae AB110/pDPkexp compared to the culture supernatants of S.cerevisiae AB110/pDP34. This 68 kDa protein reacts with the antibodies raised against a part of the yscF protein.

Example 6: In vitro processing of pre-pro-IGF1

IGF1 is expressed in S.cerevisiae as an intracellular fusion protein composed of mature IGF1 fused to the leader sequence of the S.cerevisiae α-factor precursor. The fusion protein has the following structure:

N-(α-factor leader)-IGF1-C

The amino acid sequence Tyr-Lys-Arg is the C-terminal end of the 89 amino acid α-factor leader sequence [Kurjan, J. and Herskowitz, I. 1982, Cell 30, 933-943] and a substrate for the yscF endoprotease. The fusion protein is cleaved by the truncated soluble yscF protease which leads to the digestion products N-pre-pro-α-factor-C and mature IGF1.

S.cerevisiae crude extract samples each containing 50 pmol of the α-factor leader IGF1 fusion protein are incubated with culture supematant samples of S.cerevisiae AB110/pDPkexp each containing 5 mU soluble yscF endoprotease activity (calculated from the endoprotease activity assay described in example 2). The reactions are performed in 10 µl 50 mM Na-phosphate buffer pH 7.5 for different time periods. Mature IGF1 derived from the in vitro processing procedure is detected by "Western blot" analysis. This method is described in example 5. Hybridization is performed using polyclonal antibodies raised in rabbits against mature IGF1. Detection of specific binding is described in example 5. Western blot analysis shows the processing of pre-pro-IGF1 with a culture supernatant containing the soluble yscF protease variant secreted from the S.cerevisiae AB110/pDPkexp transformant. The pre-pro-IGF1 protein is processed to the mature IGF1 protein within 30 to 120 min using the described assay conditions.

Example 7:Construction of a hybrid gene containing the GAPFL promoter and the KEX1 structural gene.

As disclosed in European Patent Application No. 341,215 the full-length KEX1 gene is isolated from total genomic Saccharomyces cerevisiae DNA on a 3.1 kb HindIII fragment. This fragment is cloned into the unique HindIII site of the yeast plasmid pJDB207 [Beggs, J.D., 1981, in D. von Wettstein et al. (ed.), Molecular Genetics in Yeast, Alfred Benzon Symposium 16. Munsgaard, Copenhagen]. E. coli HB101 is transformed with the resulting plasmid pJDB207/KEX1. The transformed E. coli strain is designated E. coli HB101/KEX1.

In order to obtain high level expression of a secreted soluble form of yscα (the protein encoded by KEX1), the very weak KEX1-promoter present on the HindIII fragment is exchanged for the strong constitutive promoter element (GAP-FL) of the yeast glyceraldehyde-3-phosphate dehydrogenase promoter (European Patent Application No. 225,633). Since the GAPFL promoter is contained on a 478 bp SalI-EcoRI fragment, first an EcoRI-site in the KEX1 coding region is eliminated by in vitro mutagenesis, followed by introduction of a new EcoRI site directly 5' to the KEX1 ATG start-codon to allow proper fusion of the GAPFL promoter to the KEX1 coding region.

In detail, the 3.1 kb HindIII fragment containing KEX1 is subcloned into the HindIII site of the M13-derived vector pBluescript KS+ (Stratagene, La Jolla, Ca. USA) to give KS+/KEX1. To remove the internal EcoRI site located 420 bp upstream of the 5'-HindIII site, the following oligonucleotide is synthesized:

5'- CCTTTTAGGGTCAATTCAGACGGT -3'

Site-directed in vitro mutagenesis is carried out as described (Bio-Rad Muta-Gene M13 kit, Bio-Rad, Richmond, Ca. USA). First, KS+/KEX1 is transfected into E. coli CJ 236 to incorporate uracil (Muta-Gene kit, supra). Single-stranded DNA from transfected E. coli CJ 236 is isolated using M13 helper phage (Stratagene, supra).

200 pmoles of the oligonucleotide primer are phosphorylated in a total volume of 30 µl containing 3 µl 1 M Tris-HCl pH 8.0, 0.3 µl 1 M MgC12, 0.75 µl 0.2 M DTT, 0.6 µl 20 mM ATP. 4.5 units T4 polynucleotide kinase are added and the mixture incubated at 37°C for 45 min and at 65°C for 10 min.

The phosphorylated oligonucleotide is then annealed to the template DNA under the following conditions: 0.1 pmoles of uracil containing DNA derived from KS+/KEX1 are incubated with 2 pmoles of phosphorylated primer in a total volume of 10 µl annealing buffer (20 mM Tris-HCl pH 7.4, 2 mM MgC12, 50 mM NaCl). The mixture is heated in a water bath to 80°C and then allowed to cool slowly until ambient temperature is reached.

The complementary strand is then formed under the following conditions: 10 µl of the annealing mixture are incubated with 4 µl 2 mM dNTP's, 0.75 µl 0.5 M Tris-HCl pH 7.4, 0.75 µl 0.1 M MgC12, 2. 15 µl 0.2 M DTT 1 unit T4 DNA polymerase and 2 units T4 DNA ligase. The reaction mixture is first incubated on ice for 5 min, then at 25°C for 5 min and finally at 37°C for 90 min. The resulting double-stranded DNA's are transformed into E. coli JM 101, a strain which efficiently removes the uracil-containing template, leaving the mutagenized complementary strand to replicate (Bio-Rad, supra). Plasmids are prepared and analysed for the absence of the EcoRI site. Correct mutagenesis is further confirmed by sequence analysis. One plasmid with the correct disruption of the internal EcoRI site is designated as KS+/KEX1 (-EcoRI).

A new EcoRI site is now introduced into KS+/KEX1(-EcoRI) using the following oligonucleotide as primer:

5'- AGATAAAGACCTGAATTCAGATGTTTTACAAT -3'

In vitro mutagenesis is carried out exactly as described supra. Plasmids are checked by restriction analysis for the presence of the new EcoRI site immediately adjacent to the start codon and by sequence analysis. One plasmid with the correct new EcoRI site is designated as KS+/KEX1 (EcoRInew).

KS+/KEX1(EcoRInew) is digested with EcoRI and HindIII and the 3.0 kb KEX1 containing fragment separated on a 0.8 % preparative agarose gel and isolated using Geneclean (Bio 101 Inc., La Jolla, CA, USA).

To obtain the GAPFL promoter fragment plasmid pJDB207/GAPFL-HIR (European Patent Application No 225,633) is digested with SalI and EcoRI and the 478 bp fragment isolated using the same procedure as described (Geneclean, supra).

0.2 pmoles of the 478 bp SalI-EcoRI promoter fragment, 0.2 pmoles of the EcoRI-HindIII fragment containg the full-length KEX1 structural gene and 0.1 pmoles of SalI-HindIII cut yeast multicopy vector pDP34 (cf. European Patent Application No. 340,170) are ligated and plasmids prepared from transformed E. coli JM 101 cells. One correct plasmid is referred to as pDP34/GAPFL-KEX1.

Example 8: Construction of a shortened KEX1 gene encoding soluble yscα activity

KEX1 encodes a membrane-bound carboxypeptidase - yscα - which is localised within the secretory machinery, most probably in the Golgi-apparatus. The yscα protein consists of an amino-terminal catalytic domain, followed by an Asp-Glu rich acidic region, a transmembrane domain and a C-terminal tail. To allow for secretion into the medium via the default pathway, truncation at the C-terminus is the method of choice. A convenient unique XhoI site is located shortly upstream of the transmembrane domain of the encoded protein at the end of the acidic region.

KS+/KEX1 (EcoRI new) is digested with EcoRI and XhoI and the 1.7 kb fragment containing the C-terminally truncated KEX1 gene isolated. This 1.7 kb KEX1 encoding fragment and the 478 bp SalI/EcoRI GAPFL promoter fragment (Example 7) are then ligated into the unique SalI site of pDP34. The resulting plasmid is referred to as pDP34/GAPFL-KEX1*. The ORF encoding soluble yscα* with KEX1-derived sequences is depicted in the sequence listing under SEQ ID No. 2.

Example 9: Transformation of S. cerevisiae strain Tr 1176

a. Crossing of S. cerevisiae kex1 mutant strain 96 with S. cerevisiae strain BYS and analysis of the spores on α-factor secretory capacity and carboxypeptidase yscα (KEX1 gene) activity

The S. cerevisiae kex1 mutant strain 96 (a, kex1, ade2, thr1), which is obtained from the yeast Genetic Stock Center, Berkeley, USA, is crossed into S. cerevisiae strain BYS232-31-42 (α, prb1-1, prc1-1, cpsl-3, lys2, leu2, his7) [Achstetter, T. and Wolf, D.H. (1985) EMBO J. 4, 173 - 177; Wolf, D.H. and Ehmann, C. (1981) J. Bacteriol. 147, 418 - 426] carrying the wild-type KEX1 allele. Diploid heterozygous cells of the genotype kex1/KEX1 are isolated from this cross. The tetrads which derive from the diploid cells are dissected according to standard genetic techniques [Hawthorne, D.C. and Mortimer, R.K. (1960) Genetics 45, 1085 - 1110; Methods in Yeast Genetics 1986 (Sherman, F. et al., eds.) Cold Spring Harbor Laboratory, N.Y.].

The four spores of each tetrad are tested for their ability to secrete α-factor. To distinguish between KEX1 wild-type and kexl mutant colonies, the pheromone-supersensitive tester strain S. cerevisiae RC629 (a, sst-2, ade2-1, ura1, his6, met1, can1, cyh2, rme) is used [Chan, R.K. and Otte, C.K. (1982) Mol. Cell. Biol. 2, 11 - 20; Chan, R.K. and Otte, C.K. (1982) Mol. Cell. Biol. 2, 21 - 29]. As expected from traits coded for by single nuclear genes, from all tetrads analysed, two spores of each tetrad secrete the a-factor, whereas the two other spores secrete α-factor. Wild-type KEX1 colonies of the α-mating type inhibit growth of the tester strain to a large extent and thus produce a large halo around themselves, since they are able to process the α-factor precursor completely and produce four active α-factor molecules from one precursor molecule. In contrast, kexl mutant colonies inhibit the growth of the tester strain to a less extent and thus produce a small halo around themselves, since they are only able to produce one mature α-factor molecule from one precursor molecule.

The spores of several complete tetrads which are identified as defective at the kexl gene by the above described pheromone assay, are finally tested for specific activity of carboxypeptidase yscα. Cells are grown, membranes thereof are prepared and tested for carboxypeptidase yscα activity using Cbz-Tyr-Lys-Arg as substrate as described [Wagner, J.C. and Wolf, D.H. (1987) FEBS Lett. 221, 2, 423 - 426]. The fact that activity of carboxypeptidase yscα is lacking in kexl mutant cells, indicates that KEX1 is the structural gene of this enzyme. This implies that carboxypeptidase yscα is indeed involved in carboxy-terminal processing of α-factor.

b. Classification of confirmed kex 1 mutants on additional deficiency of proteases yscB, yscY and yscS

S. cerevisiae kex1 mutants are classified with regard to the deficiency of other proteases (proteinase yscB, carboxypeptidase yscY and carboxy-peptidase yscS) and additional growth factor requirements.

Cell material of kex1 mutants which are prepared from stationary phase in YPD (Difco) medium is suspended in the 200 µl 20 mM Tris-HCl buffer, pH 7.2 in Eppendorf microfuge and glass beads (0.4 mm in diameter) are added up to two thirds of the volume. The suspension is heavily shaken three times for 1 min on a vortex mixer with intermittent cooling on ice.

Centrifugation for 5 min allows recovery of the supernatant crude extracts. These extracts are dialysed against 0.1 M imidazole-HCl buffer pH 5.2 with 0.1 mM $ZnCl_2$ in order to activate proteases and to remove free amino acids from the extracts.

Proteinase yscB activities are measured according to the Azocoll- test [R.E. Ulane et al. (1976) J. Biol. Chem. 251, 3367; E. Cabib et al. (1973) Biochem. Biophys. Res. Commun. 50, 186; T. Saheki et al. (1974) Eur. J. Biochem. 42, 621]. After the protein concentration measurements, an aliquot of each sample is filled with 0.1 M sodium phosphate (NaPi) buffer pH 7.0 up to 100 µl to adjust the required equal protein amounts. To the protein solution, a suspension of 500 µl Azocoll (240 mg in 10 ml 0.1 M NaPi buffer, pH 7.0) is added. These mixtures are incubated at 30°C for one hour with agitation. After the addition of 500 µl 10% tri-chloroacetic acid which stops the reaction, the mixtures are centrifuged two times and the absorption spectra of the supernatants at 520 nm are measured.

The activities of carboxypeptidase yscY and yscS are measured using the chromogenic substrate Cbz-Gln-Leu [cf. D.H. Wolf et al. (1978) FEBS Lett. 91, 59; D.H. Wolf et al. (1977) Eur.J. Biochem. 73, 553]. The dialysed extracts are divided into three portions and to two of them phenyl-methylsulfonyl fluoride (PMSF) at a final concentration of 1 mM or EDTA at a final concentration of 5 mM is added to block the two protease activities selectively. Namely PMSF inhibits carboxypeptidase yscY activity and EDTA inhibits that of carboxypeptidase yscS. The mixtures with inhibitors are each incubated at 25°C for one hour to complete the inhibition. After the determination of the protein concentration, two aliquots with inhibitor and one aliquot without inhibitor as a control of each sample are filled with 0.1 M NaPi buffer pH 7.4 up to 50 µl in order to receive equal protein amounts. To these protein solutions the following test solutions are added.

500 µl test solution I:

| | |
|---|---|
| L-amino acid oxidase<br>horseradish peroxidase<br>0.01 mM $MnCl_2$<br>in 0.1 M NaPi buffer, pH 7.4 | 0.24 mg/ml<br>0.40 mg/ml |

50 µl test solution II

| | |
|---|---|
| o-dianisidin<br>in water | 2 mg/ml |

500 µl test solution III
20 mM Cbz-Gly-Leu
in 0.2 M potassium phosphate buffer, pH 7.4

The mixtures are incubated at 28°C for one hour and after the addition of 100 µl 20 % Triton X-100 to stop the reaction, the absorbances at 405 nm are measured.

For the purpose of the subsequent transformation, an amino acid auxotrophic marker for leucine is scored with the replica-technique on minimal plates supplied with adenine, threonine, lysine and histidine, and with or without leucine.

By means of the above described assays, mutants are isolated designated S. cerevisiae BYskex1, which exhibit a quadruple protease-deficiency (α, prb-1, prc-1, cps-3, kex1) and an additional requirement for leucine.

c. Production of ura3-deficient yeast strain TR1176

Tr 1176 is a ura3- derivative of strain BYSkex1 described above. Disruption of the URA3 gene in BYSkex1 is carried out as detailed in European Patent Application Nr. 340,170. Tr 1176 has the following genetic markers: MATα, prb-1, prc-1, cps-1, kex-1, ade2, leu2, ura3.

S. cerevisiae Tr 1176 is transformed with plasmids pDP34/GAPFL-KEX1 and pDP34/GAPFL-KEX1* using the transformation protocol described by Dohmen (supra). Transformed yeast cells are selected on yeast minimal media plates supplemented with leucine and adenine and deficient in uracil. Single transformed yeast colonies are isolated and referred to as:

Saccharomyces cerevisiae Tr 1176/pDP34/GAPFL-KEX1 and
Saccharomyces cerevisiae Tr 1176/pDP34/GAPFL-KEX1*

Example 10: Biological activity of soluble yscα

Cells of Saccharomyces cerevisiae Tr 1176/pDP34/GAPFL-KEX1 and Saccharomyces cerevisiae Tr 1176/pDP34/GAPFL-KEX1* are grown in minimal medium composed of:

| | |
|---|---|
| Difco Yeast Nitrogen Base w/o amino acids | 8.4 g/l glucose<br>20.0 g/l L-asparagine<br>10.0 g/l adenine<br>0.1 g/l |

for 48 h at 30°C and 180 r.p.m.

The cells are separated from the medium by centrifugation, cells are resuspended in 0.9 % NaCl and tested for yscα activity by halo-formation on a lawn of α-factor supersensitive a-cells (Chan, R.K. and Otte, C.K. (1982) Mol.Cell. Biol.2, 11-20). Strain Tr 1176 without plasmid shows only a minute halo on the a-cell lawn due to the lack of yscα activity. Saccharomyces cerevisiae Tr 1176/pDP34/GAPFL-KEX1 and Saccharomyces cerevisiae Tr 1176/pDP34/GAPFL-KEX1* both show halo-formation, soluble yscα has therefore retained its normal biological function, i.e. α-factor maturation.

Supernatants of Saccharomyces cerevisiae Tr 1176/pDP34/GAPFL-KEX1 and Saccharomyces cerevisiae Tr 1176/pDP34/GAPFL-KEX1* fermentations (supra) are then analysed for the presence of yscα activity in the medium using the synthetic peptide substrate Cbz-Tyr-Lys-Arg and an assay as described (Wolf, D.H. and Weiser,U. (1977) Eur.J.Biochem. 73, 553-556). Only supernatants of Saccharomyces cerevisiae Tr 1176/pDP34/GAPFL-KEX1* show proteolytic degradation of this substrate, whereas supernatants of Saccharomyces cerevisiae Tr 1176/pDP34/GAPFL-KEX1 have no activity on Cbz-Tyr-Lys-Arg. It can therefore be concluded that KEX1* encodes biologically active yscα which is released into the medium. The new protein is referred to as yscα*.

Example 11: Purification of yscα*

5 ml of the ion exchanger Fractogel TSK DEAE 650 (Merck AG, Darmstadt, FRG) are packed into a column and equilibrated with 50 mM sodium phosphate buffer, pH 7.0. 80 ml of culture broth of Saccharomyces cerevisiae Tr 1176/pDP34/GAPFL-KEX1* (supra) are adjusted to pH 7.0 and loaded onto the column overnight yscα* under those conditions binds to the column. Bound proteins are then eluted with a combined pH/salt gradient (40 ml 50 mM sodium phoshate buffer pH 7.0 and 40 ml 50 mM sodium-phosphate buffer pH 4.0 + 1 M NaCl). At about 0.2 M NaCl yscα* elutes in essentially pure form with an apparent molecular weight of about 66 kDa (calculated molecular weight 65 kDa). When compared with porcine pancreas carboxypeptidase B (Boehringer Mannheim, Mannheim, FRG) yscα* at the same protein concentration has the equivalent activity on the substrate Cbz-Tyr-Lys-Arg at pH 7.0 and slightly higher activity at pH 4.0.

Example 12: Construction of a recombinant fusion peptide expressing hirudin and human calcitonin simultaneously

A fusion peptide is constructed which allows for simultaneous expression of hirudin and of precursor human calcitonin. Between the two peptides the KEX2/KEX1 recognition site Lys-Arg is introduced to allow for in vitro processing with soluble yscF and soluble yscα* (supra).

In detail, plasmid pJDB207/GAPFL-HIR (supra, Example 7) is digested with SalI and BamHI and the 0.7 kb fragment subcloned into SalI-BamHI of pBluescript KS$^+$. The gene encoding the C-terminal glycine-precursor of human calcitonin is assembled from individual oligonucleotides as described for hirudin (cf. European Patent Application No. 168342) and subcloned into the PstI site of pUC19. The sequence of human precursor calcitonin with a 5'-extension to provide for in vitro processing is depicted in the sequence listing under SEQ ID No. 3 (as confirmed by sequence analysis).

The 132 bp PstI fragment is excised and cloned into the PstI site of the hirudin containing Bluescript vector.

Transformants are checked for proper orientation of the insert and one correct plasmid is referred to as KS+/GAPFL-HIR-CALC.

Plasmid pJDB207/GAPFL-HIR is digested with SalI and HindIII and the 7 kb large vector fragment isolated. Plasmid pJDB207/GAPFL-HIR is digested with BamHI and HindIII to obtain the 350 bp PH05 terminator fragment.

Finally, pJDB207 vector fragment, terminator fragment and the SalI-BamHI fragment of KS+/GAPFL-HIR-CALC are ligated in a triple ligation to yield plasmid pJDB207/GAPFL-HIR-CALC.

Saccharomyces cerevisiae strain HT246 (DSM 4084) is transformed with plasmid pJDB207/GAPFL-HIR-CALC according to Example 3. Transformed yeast cells are selected on yeast minimal medium plates deficient in leucine. Single transformed yeast cells are isolated and referred to as S. cerevisiae HT246/pJDB207/GAPFL-HIR-CALC.

For the expression and secretion of the fusion protein S. cerevisiae HT246/pJDB207/GAPFL-HIR-CALC is cultivated as disclosed in European Patent Application No. 340170 (example 10 therein). After 48h and 72h of cultivation samples are withdrawn, the cells removed by centrifugation and the culture supernatant containing the fusion protein taken for further processing. First, the supernatant containing the hirudin-calcitonin fusion protein is endoproteolytically split by digestion with truncated soluble yscF protein according to Example 6. This cleavage results in full-length precursor human calcitonin and a hirudin variant which still contains a lysine-arginine extension at its C-terminus. This lysine-arginine extension is removed by digestion with yscα* using purified enzyme (see Example 11). Correct removal of the lysine-arginine extension is demonstrated by reversed phase HPLC of the reaction mixture as disclosed in European Patent Application No. 340170.

Example 13: Construction of a recombinant fusion protein expressing eglin C

A fusion peptide is constructed which allows the expression of a fusion protein according to the formula P-L-T in which P is the eglin C polypeptide (Rink H. et al., 1984 Nucl. Acids Res. 12, 6369-6387), L the linker sequence **Lys-Arg-Glu-Ala-Glu-Ala-Trp-Val-Pro** and T a cellulose binding domain of the Cellulomonas fimi Exg protein encoded in the Cellulomonas fimi cex gene (Neill G.O. et al., 1986 Gene 44, 325-330).

In detail, plasmid pEC-1 (Neill G.O. et al., 1986 Gene 44, 325-330) is cut to completion with SmaI and StuI. The 433 bp fragment encoding the cellulose binding domain of the cellulomonas cex gene is isolated. Plasmid pl8kexp (example 1) is cut with Asp718 and the sticky ends are filled in using Klenow polymerase resulting in blunt ends. The linearised plasmid is ligated with the 433 bp SmaI-StuI cex fragment and transformed into E.coli HB101. The clones are checked for the orientation of the 433 bp cex fragment. One clone with the orientation that couples the reading frame of the KEX2 gene to the reading frame of the cex gene is referred to as p18kexpcex.

Plasmid pUC19 is cut with SalI and BamHI and the 276 bp SalI-BamHI fragment of pBR322 is inserted. The resulting plasmid pUC19pBR is cut with BamHI and EcoRI and ligated in the presence of the BamHI-EcoRI GAPDH promoter fragment resulting in plasmid pTM1. pTM 1 is cut with EcoRI and the following linker is inserted

5'-AATTCATGCA TGCATGCAGA TCT-3'
3'-GTACGT ACGTACGTCT AGATTAA-5'

in such a way that the EcoRI site is reconstituted adjacent to the promoter sequences. The resulting plasmid is referred to as pTM2.

Plasmid pML147 (Rink H. et al., 1984 Nucl. Acids Res. 12, 6369-6387) is cut to completion with EcoRI and BamHI. The EcoRI-BamHI eglin C fragment is cloned into EcoRI/BamHI cut pBR322. One plasmid with the desired sequence is called pML141. Using the oligonucleotides

5'-CCGGTACCCA AGCTTCGGCT TCTCTCTTAC CAACATGCGG AACATG-3'

and

5'-CGGGATCCAA GAATTCATGA CTGAATT-3'

(the sequence coding for the linker sequence L referred to above is underlined) a PCR reaction is performed on pML141 using the PCR-Kit from Perkin- Elmer-Cetus and following strictly the instructions of the supplier. The PCR reaction results in the amplification of a 257 bp fragment containing the eglin C gene joined to the linker sequence L. The fragment is isolated from an agarose gel and cut to completion with BamHI and Asp718. Plasmid pl8kexpcex (see above) is cut to completion with BamHI and Asp718 and the 3.1 kb fragment is isolated. The 3.1 kb BamHI-Asp718 p18kexpcex fragment containing the cex gene fragment and pUC19 sequences is ligated with the PCR generated BamHI-Asp718 eglin C fragment and the resulting plasmid is called p18eglincex. The plasmid contains an open reading frame reaching from the first amino acid of eglin C to the last amino acid of the Exg protein. The two domains, i.e. eglin C and the 133 C-terminal amino acids of Exg (including an extra proline), are separated by the linker sequence given above. The nucleotide sequence encoding the fusion protein (see SEQ ID No. 4) is confirmed by DNA seqencing.

Plasmid pTM2 (see above) is cut with EcoRI and the 5'-ends are dephosphorylated. Plasmid p18eglincex is cut with EcoRI and the 686 bp eglin-cex fragment is isolated. This 686 bp fragment is ligated into the EcoRI cut and dephosphorylated vector pTM2. The resulting clones are checked for the orientation of the eglin-cex fragment. One clone with the desired orientation resulting in the following order of the sequences GAPDH-eglin-cex is called p19/GAPDH-EGLIN-CEX.

Plasmid p19/GAPDH-EGLIN-CEX is digested with BamHI and Bg1II and the 1.1 kb fragment encoding the GAPDH-eglin-cex sequences is isolated. Plasmid pDP34 is cut with BamHI, the 5'-ends are dephosphorylated and the resulting fragment is ligated with the 1.1 kb BamHI-BglII fragment. The clones are checked for the orientation of the 1.1 kb BamHI-BglII fragment and referred to as pDP34GAPDH-eglincex-1 and pDP34GAPDH-eglincex-2 wherein index 1 indicates that the insert GAPDH-eglin-cex is clockwise oriented in plasmid pDP34 while index 2 indicates a counterclockwise orientation.

pDP34GAPDH-eglincex-1 is transformed into Saccharomyces cerevisiae strain AB110 (ATCC 20796) according to example 3. Cells of AB110/pDP34GAPDH-eglincex-1 are grown in 10 ml minimal medium (Difco Yeast Nitrogen Base w/o Amino Acids 6.7 gl, glucose 40 g/l, amino acid/nucleotide mix according to example 3 w/o leucine) for 24 h at 30 C with 180 rpm. The main cultures, 250 ml of minimal medium are inoculated with the preculture and grown at 30 C for 24 h with 180 rpm. The production culture is set up as follows: The cells of the main culture are separated from the culture broth by centrifugation, resuspended in 250 ml of double minimal medium (Difco Yeast Nitrogen Base w/o Amino Acids 13.4 g/l, glucose 40 g/l, 2x amino acid/nucleotide mix according to example 3 w/o leucine) and fermented at 30 C for 24 h with 180 rpm.

The cells of the production culture are separated from the culture medium by centrifugation, washed first in 200ml and then in 40 ml buffer A (buffer A: 50 mM Tris-HCl pH 7.0, 100 mM NaCl). Cells are broken with glass beads in repeated steps each of which in about 10 ml buffer A and the crude extract of a total volume of 40 ml is cleared by centrifugation (25000 x g, 15 min, 4 C). 5 g (dry weight) cellulose (Avicel or fibrous cellulose) is added to the crude extract and incubated on a turning shaker for 1.5 h at 4 C.

The cellulose bound fusion protein is eluted with water as a fusion protein and afterwards digested with yscF resulting in eglin C with the C-terminal extension Lys-Arg. These two C-terminal amino acids are removed by digestion with yscα. In detail: The cellulose after incubation in the crude extract is filled into a chromatography column (1.6 x 20 cm) and washed on the column with three column bed volumes buffer A. Then a gradient of buffer A and water is applied. The gradient is linear and runs from 100% buffer A, 0% water to 0% buffer A, 100 % water within three column bed volumes. Fractions are collected and tested for the fusion protein by the immunological method as described in example 5. Fractions containing the fusion protein are subjected to a treatment with the soluble protease yscF as described in example 6 resulting in the cleavage product eglinC-Lys-Arg which is afterwards digested with soluble yscα* to remove the Lys-Arg tail.

Alternatively, the cellulose bound fusion protein is digested with yscF while bound to cellulose and a protein consisting of eglin C with the C-terminal extension Lys-Arg is set free. This protein is afterwards digested with yscα* to remove these two C-terminal amino acids. In detail: Different amounts (10 mg to 500 mg) of the cellulose after incubation with the crude extract are incubated with the soluble protease yscF as described in example 6 whereby eglinC-Lys-Arg is set free. Eglin C-Lys-Arg is further digested with soluble yscα* in order to remove the Lys-Arg tail. The obtained eglin C is identical in structure to natural eglin C as evidenced by HPLC.

Deposition of microorganisms

The following microorganism strains were deposited at the Deutsche Sammlung von Mikroorganismen (DSM), Mascheroder Weg 1b, D-3300 Braunschweig (deposition dates and accession numbers given):

Escherichia coli JM109/pDP34: March 14, 1988, DSM 4473.
Saccharomyces cerevisiae BYS232-31-42: May 6, 1988, DSM 4583.
Escherichia coli JM101/pKS301b: June 25, 1990, DSM 6028.
Escherichia coli HB 101/KEX1: June 25, 1990, DSM 6027.
Escherichia coli JM101/p19/GAPDH-EGLIN-CEX: June 3, 1991, DSM6546.

**SEQ ID No. 1**

Sequence type: Polynucleotide with corresponding polypeptide
Sequence length: 1866 base pairs
Strandedness: double
Topology: linear
Source: yeast genomic DNA
Immediate experimental source: E.coli JM101/pKS301b (DSM6028)
Features: from 1 to 1866 coding region for soluble yscF

```
ATG AAA GTG AGG AAA TAT ATT ACT TTA TGC TTT TGG TGG     39
Met Lys Val Arg Lys Tyr Ile Thr Leu Cys Phe Trp Trp
  1               5                  10

GCC TTT TCA ACA TCC GCT CTT GTA TCA TCA CAA CAA ATT     78
Ala Phe Ser Thr Ser Ala Leu Val Ser Ser Gln Gln Ile
     15                  20              25

CCA TTG AAG GAC CAT ACG TCA CGA CAG TAT TTT GCT GTA    117
Pro Leu Lys Asp His Thr Ser Arg Gln Tyr Phe Ala Val
                30                  35

GAA AGC AAT GAA ACA TTA TCC CGC TTG GAG GAA ATG CAT    156
Glu Ser Asn Glu Thr Leu Ser Arg Leu Glu Glu Met His
40                      45                  50

CCA AAT TGG AAA TAT GAA CAT GAT GTT CGA GGG CTA CCA    195
Pro Asn Trp Lys Tyr Glu His Asp Val Arg Gly Leu Pro
        55                  60                  65

AAC CAT TAT GTT TTT TCA AAA GAG TTG CTA AAA TTG GGC    234
Asn His Tyr Val Phe Ser Lys Glu Leu Leu Lys Leu Gly
                    70                  75

AAA AGA TCA TCA TTA GAA GAG TTA CAG GGG GAT AAC AAC    279
Lys Arg Ser Ser Leu Glu Glu Leu Gln Gly Asp Asn Asn
     80                  85                  90
```

```
GAC CAC ATA TTA TCT GTC CAT GAT TTA TTC CCG CGT AAC    312
Asp His Ile Leu Ser Val His Asp Leu Phe Pro Arg Asn
                 95                 100

GAC CTA TTT AAG AGA CTA CCG GTG CCT GCT CCA CCA ATG    351
Asp Leu Phe Lys Arg Leu Pro Val Pro Ala Pro Pro Met
105                 110                 115

GAC TCA AGC TTG TTA CCG GTA AAA GAA GCT GAG GAT AAA    390
Asp Ser Ser Leu Leu Pro Val Lys Glu Ala Glu Asp Lys
        120                 125                 130

CTC AGC ATA AAT GAT CCG CTT TTT GAG AGG CAG TGG CAC    429
Leu Ser Ile Asn Asp Pro Leu Phe Glu Arg Gln Trp His
                135                 140

TTG GTC AAT CCA AGT TTT CCT GGC AGT GAT ATA AAT GTT    468
Leu Val Asn Pro Ser Phe Pro Gly Ser Asp Ile Asn Val
    145                 150                 155

CTT GAT CTG TGG TAC AAT AAT ATT ACA GGC GCA GGG GTC    507
Leu Asp Leu Trp Tyr Asn Asn Ile Thr Gly Ala Gly Val
            160                 165

GTG GCT GCC ATT GTT GAT GAT GGC CTT GAC TAC GAA AAT    546
Val Ala Ala Ile Val Asp Asp Gly Leu Asp Tyr Glu Asn
170                 175                 180

GAA GAC TTG AAG GAT AAT TTT TGC GCT GAA GGT TCT TGG    585
Glu Asp Leu Lys Asp Asn Phe Cys Ala Glu Gly Ser Trp
        185                 190                 195

GAT TTC AAC GAC AAT ACC AAT TTA CCT AAA CCA AGA TTA    624
Asp Phe Asn Asp Asn Thr Asn Leu Pro Lys Pro Arg Leu
                200                 205
```

```
TCT GAT GAC TAC CAT GGT ACG AGA TGT GCA GGT GAA ATA   663
Ser Asp Asp Tyr His Gly Thr Arg Cys Ala Gly Glu Ile
    210                 215                 220

GCT GCC AAA AAA GGT AAC AAT TTT TGC GGT GTC GGG GTA   702
Ala Ala Lys Lys Gly Asn Asn Phe Cys Gly Val Gly Val
            225                 230

GGT TAC AAC GCT AAA ATC TCA GGC ATA AGA ATC TTA TCC   741
Gly Tyr Asn Ala Lys Ile Ser Gly Ile Arg Ile Leu Ser
235                 240                 245

GGT GAT ATC ACT ACG GAA GAT GAA GCT GCG TCC TTG ATT   780
Gly Asp Ile Thr Thr Glu Asp Glu Ala Ala Ser Leu Ile
        250                 255                 260

TAT GGT CTA GAC GTA AAC GAT ATA TAT TCA TGC TCA TGG   819
Tyr Gly Leu Asp Val Asn Asp Ile Tyr Ser Cys Ser Trp
                265                 270

GGT CCC GCT GAT GAC GGA AGA CAT TTA CAA GGC CCT AGT   858
Gly Pro Ala Asp Asp Gly Arg His Leu Gln Gly Pro Ser
    275                 280                 285

GAC CTG GTG AAA AAG GCT TTA GTA AAA GGT GTT ACT GAG   897
Asp Leu Val Lys Lys Ala Leu Val Lys Gly Val Thr Glu
            290                 295

GGA AGA GAT TCC AAA GGA GCG ATT TAC GTT TTT GCC AGT   936
Gly Arg Asp Ser Lys Gly Ala Ile Tyr Val Phe Ala Ser
300                 305                 310

GGA AAT GGT GGA ACT CGT GGT GAT AAT TGC AAT TAC GAC   975
Gly Asn Gly Gly Thr Arg Gly Asp Asn Cys Asn Tyr Asp
        315                 320                 325
```

```
GGC TAT ACT AAT TCC ATA TAT TCT ATT ACT ATT GGG GCT  1014
Gly Tyr Thr Asn Ser Ile Tyr Ser Ile Thr Ile Gly Ala
                330                 335

ATT GAT CAC AAA GAT CTA CAT CCT CCT TAT TCC GAA GGT  1053
Ile Asp His Lys Asp Leu His Pro Pro Tyr Ser Glu Gly
    340                 345                 350

TGT TCC GCC GTC ATG GCA GTC ACG TAT TCT TCA GGT TCA  1092
Cys Ser Ala Val Met Ala Val Thr Tyr Ser Ser Gly Ser
            355                 360

GGC GAA TAT ATT CAT TCG AGT GAT ATC AAC GGC AGA TGC  1131
Gly Glu Tyr Ile His Ser Ser Asp Ile Asn Gly Arg Cys
365                 370                 375

AGT AAT AGC CAC GGT GGA ACG TCT GCG GCT GCT CCA TTA  1170
Ser Asn Ser His Gly Gly Thr Ser Ala Ala Ala Pro Leu
        380                 385                 390

GCT GCC GGT GTT TAC ACT TTG TTA CTA GAA GCC AAC CCA  1209
Ala Ala Gly Val Tyr Thr Leu Leu Leu Glu Ala Asn Pro
                395                 400

AAC CTA ACT TGG AGA GAC GTA CAG TAT TTA TCA ATC TTG  1248
Asn Leu Thr Trp Arg Asp Val Gln Tyr Leu Ser Ile Leu
    405                 410                 415

TCT GCG GTA GGG TTA GAA AAG AAC GCT GAC GGA GAT TGG  1287
Ser Ala Val Gly Leu Glu Lys Asn Ala Asp Gly Asp Trp
            420                 425

AGA GAT AGC GCC ATG GGG AAG AAA TAC TCT CAT CGC TAT  1326
Arg Asp Ser Ala Met Gly Lys Lys Tyr Ser His Arg Tyr
430                 435                 440
```

```
GGC TTT GGT AAA ATC GAT GCC CAT AAG TTA ATT GAA ATG  1365
Gly Phe Gly Lys Ile Asp Ala His Lys Leu Ile Glu Met
        445                 450                 455

TCC AAG ACC TGG GAG AAT GTT AAC GCA CAA ACC TGG TTT  1404
Ser Lys Thr Trp Glu Asn Val Asn Ala Gln Thr Trp Phe
                460                 465

TAC CTG CCA ACA TTG TAT GTT TCC CAG TCC ACA AAC TCC  1449
Tyr Leu Pro Thr Leu Tyr Val Ser Gln Ser Thr Asn Ser
    470                 475                 480

ACG GAA GAG ACA TTA GAA TCC GTC ATA ACC ATA TCA GAA  1482
Thr Glu Glu Thr Leu Glu Ser Val Ile Thr Ile Ser Glu
            485                 490

AAA AGT CTT CAA GAT GCT AAC TTC AAG AGA ATT GAG CAC  1521
Lys Ser Leu Gln Asp Ala Asn Phe Lys Arg Ile Glu His
495                 500                 505

GTC ACG GTA ACT GTA GAT ATT GAT ACA GAA ATT AGG GGA  1560
Val Thr Val Thr Val Asp Ile Asp Thr Glu Ile Arg Gly
        510                 515                 520

ACT ACG ACT GTC GAT TTA ATA TCA CCA GCG GGG ATA ATT  1599
Thr Thr Thr Val Asp Leu Ile Ser Pro Ala Gly Ile Ile
                525                 530

TCA AAC CTT GGC GTT GTA AGA CCA AGA GAT GTT TCA TCA  1638
Ser Asn Leu Gly Val Val Arg Pro Arg Asp Val Ser Ser
    535                 540                 545

GAG GGA TTC AAA GAC TGG ACA TTC ATG TCT GTA GCA CAT  1677
Glu Gly Phe Lys Asp Trp Thr Phe Met Ser Val Ala His
            550                 555
```

```
TGG GGT GAG AAC GGC GTA GGT GAT TGG AAA ATC AAG GTT  1716
Trp Gly Glu Asn Gly Val Gly Asp Trp Lys Ile Lys Val
560                 565                 570

AAG ACA ACA GAA AAT GGA CAC AGG ATT GAC TTC CAC AGT  1755
Lys Thr Thr Glu Asn Gly His Arg Ile Asp Phe His Ser
        575                 580                 585

TGG AGG CTG AAG CTC TTT GGG GAA TCC ATT GAT TCA TCT  1794
Trp Arg Leu Lys Leu Phe Gly Glu Ser Ile Asp Ser Ser
                590                 595

AAA ACA GAA ACT TTC GTC TTT GGA AAC GAT AAA GAG GAG  1833
Lys Thr Glu Thr Phe Val Phe Gly Asn Asp Lys Glu Glu
    600                 605                 610

GTT GAA CCA GGG GTA CCG AGC TCG AAT TCG TAA          1866
Val Glu Pro Gly Val Pro Ser Ser Asn Ser
            615                 620
```

**SEQ ID No. 2**

Sequence type: Polynucleotide with corresponding polypeptide

Sequence length: 1797 base pairs

Strandedness: double

Topology: linear

Source: yeast genomic DNA

Immediate experimental source: E.coli HB101/KEX1 (DSM6027) Features: From 1 to 1797 coding region for soluble yscα*

```
ATG TTT TAC AAT AGG TGG CTC GGA ACG TGG CTA GCC ATG     39
Met Phe Tyr Asn Arg Trp Leu Gly Thr Trp Leu Ala Met
  1               5                  10

TCT GCT TTA ATA AGG ATC TCA GTA TCC CTT CCG TCA TCT     78
Ser Ala Leu Ile Arg Ile Ser Val Ser Leu Pro Ser Ser
     15                  20                  25

GAG GAG TAC AAA GTG GCA TAT GAG CTG TTG CCA GGG TTA    117
Glu Glu Tyr Lys Val Ala Tyr Glu Leu Leu Pro Gly Leu
                30                  35

TCT GAA GTG CCA GAC CCT TCA AAT ATT CCA CAG ATG CAT    156
Ser Glu Val Pro Asp Pro Ser Asn Ile Pro Gln Met His
 40                  45                  50

GCT GGC CAT ATT CCT TTA CGT TCC GAA GAT GCG GAT GAA    195
Ala Gly His Ile Pro Leu Arg Ser Glu Asp Ala Asp Glu
          55                  60                  65

CAG GAT AGC TCT GAC TTG GAG TAC TTT TTT TGG AAG TTT    234
Gln Asp Ser Ser Asp Leu Glu Tyr Phe Phe Trp Lys Phe
                    70                  75

ACC AAT AAC GAC TCT AAT GGC AAT GTC GAC CGT CCC TTA    279
Thr Asn Asn Asp Ser Asn Gly Asn Val Asp Arg Pro Leu
     80                  85                  90
```

```
ATT ATA TGG TTA  AA GGT GGA CCC GGT TGC TCT TCC ATG    312
Ile Ile Trp Leu Asn Gly Gly Pro Gly Cys Ser Ser Met
                 95                  100

GAT GGT GCC TTG GTC GAA TCC GGC CCT TTT AGG GTG AAT    351
Asp Gly Ala Leu Val Glu Ser Gly Pro Phe Arg Val Asn
105                 110                 115

TCA GAC GGT AAA CTT TAT CTA AAT GAA GGG TCC TGG ATA    390
Ser Asp Gly Lys Leu Tyr Leu Asn Glu Gly Ser Trp Ile
        120                 125                 130

TCC AAA GGC GAT CTT TTA TTT ATC GAC CAA CCT ACT GGT    429
Ser Lys Gly Asp Leu Leu Phe Ile Asp Gln Pro Thr Gly
                    135                 140

ACT GGG TTT TCT GTC GAA CAA AAT AAA GAC GAA GGT AAA    468
Thr Gly Phe Ser Val Glu Gln Asn Lys Asp Glu Gly Lys
    145                 150                 155

ATC GAC AAA AAC AAA TTT GAC GAA GAC CTA GAA GAT GTG    507
Ile Asp Lys Asn Lys Phe Asp Glu Asp Leu Glu Asp Val
                160                 165

ACC AAG CAT TTT ATG GAT TTT CTG GAG AAC TAT TTC AAG    546
Thr Lys His Phe Met Asp Phe Leu Glu Asn Tyr Phe Lys
170                 175                 180

ATT TTT CCA GAA GAC CTC ACT AGG AAA ATC ATA CTA TCG    585
Ile Phe Pro Glu Asp Leu Thr Arg Lys Ile Ile Leu Ser
        185                 190                 195

GGT GAA AGT TAC GCT GGC CAA TAC ATA CCA TTC TTT GCC    624
Gly Glu Ser Tyr Ala Gly Gln Tyr Ile Pro Phe Phe Ala
                    200                 205
```

```
AAT GCA ATT TTG AAC CAT AAC AAA TTT TCA AAG ATC GAC    663
Asn Ala Ile Leu Asn His Asn Lys Phe Ser Lys Ile Asp
    210                 215                 220

GGG GAT ACA TAC GAC TTG AAG GCG CTA TTG ATT GGT AAC    702
Gly Asp Thr Tyr Asp Leu Lys Ala Leu Leu Ile Gly Asn
            225                 230

GGT TGG ATT GAC CCC AAT ACA CAG TCC CTA TCG TAC CTT    741
Gly Trp Ile Asp Pro Asn Thr Gln Ser Leu Ser Tyr Leu
235                 240                 245

CCG TTT GCT ATG GAG AAG AAA CTG ATT GAT GAA AGC AAC    780
Pro Phe Ala Met Glu Lys Lys Leu Ile Asp Glu Ser Asn
        250                 255                 260

CCC AAT TTC AAA CAC TTA ACG AAC GCA CAC GAG AAT TGC    819
Pro Asn Phe Lys His Leu Thr Asn Ala His Glu Asn Cys
                265                 270

CAG AAT CTG ATA AAC TCT GCC AGT ACA GAT GAG GCA GCC    858
Gln Asn Leu Ile Asn Ser Ala Ser Thr Asp Glu Ala Ala
    275                 280                 285

CAT TTT TCG TAT CAG GAA TGT GAG AAT ATT TTA AAC CTT    897
His Phe Ser Tyr Gln Glu Cys Glu Asn Ile Leu Asn Leu
            290                 295

CTA TTG TCT TAT ACC AGG GAA TCT TCG CAA AAG GGA ACA    936
Leu Leu Ser Tyr Thr Arg Glu Ser Ser Gln Lys Gly Thr
300                 305                 310

GCG GAT TGC TTG AAC ATG TAT AAC TTC AAT TTA AAA GAT    975
Ala Asp Cys Leu Asn Met Tyr Asn Phe Asn Leu Lys Asp
        315                 320                 325
```

```
AGT TAT CCA TCT TGT GGT ATG AAT TGG CCG AAA GAT ATT  1014
Ser Tyr Pro Ser Cys Gly Met Asn Trp Pro Lys Asp Ile
                    330                 335

TCC TTT GTC AGT AAA TTC TTC AGC ACA CCT GGT GTT ATT  1053
Ser Phe Val Ser Lys Phe Phe Ser Thr Pro Gly Val Ile
    340                 345                 350

GAT TCG TTG CAT CTT GAT TCT GAT AAA ATT GAT CAT TGG  1092
Asp Ser Leu His Leu Asp Ser Asp Lys Ile Asp His Trp
                355                 360

AAG GAA TGC ACT AAT AGC GTT GGA ACT AAA TTG TCT AAT  1131
Lys Glu Cys Thr Asn Ser Val Gly Thr Lys Leu Ser Asn
365                 370                 375

CCT ATT TCA AAG CCA TCT ATC CAT TTA TTA CCT GGT CTA  1170
Pro Ile Ser Lys Pro Ser Ile His Leu Leu Pro Gly Leu
        380                 385                 390

CTT GAA AGT GGA ATA GAG ATT GTC TTG TTC AAT GGT GAC  1209
Leu Glu Ser Gly Ile Glu Ile Val Leu Phe Asn Gly Asp
                    395                 400

AAA GAC TTG ATT TGT AAT AAC AAG GGC GTA TTA GAT ACT  1248
Lys Asp Leu Ile Cys Asn Asn Lys Gly Val Leu Asp Thr
    405                 410                 415

ATA GAC AAT CTA AAA TGG GGT GGA ATA AAG GGA TTT AGC  1287
Ile Asp Asn Leu Lys Trp Gly Gly Ile Lys Gly Phe Ser
                420                 425

GAC GAT GCT GTT TCG TTC GAT TGG ATC CAT AAA TCG AAG  1326
Asp Asp Ala Val Ser Phe Asp Trp Ile His Lys Ser Lys
430                 435                 440
```

```
AGT ACA GAC GAC AGC GAA GAA TTT AGC GGA TAC GTG AAG  1365
Ser Thr Asp Asp Ser Glu Glu Phe Ser Gly Tyr Val Lys
        445                 450                 455

TAT GAT AGA AAT TTG ACG TTT GTT AGC GTT TAT AAT GCT  1404
Tyr Asp Arg Asn Leu Thr Phe Val Ser Val Tyr Asn Ala
                460                 465

TCT CAC ATG GTA CCC TTC GAT AAA AGT TTA GTG AGT AGA  1443
Ser His Met Val Pro Phe Asp Lys Ser Leu Val Ser Arg
    470                 475                 480

GGC ATT GTC GAT ATT TAC TCG AAC GAT GTT ATG ATC ATT  1482
Gly Ile Val Asp Ile Tyr Ser Asn Asp Val Met Ile Ile
            485                 490

GAC AAC AAT GGG AAA AAT GTT ATG ATT ACT ACT GAC GAC  1521
Asp Asn Asn Gly Lys Asn Val Met Ile Thr Thr Asp Asp
495                 500                 505

GAT AGT GAT CAA GAT GCT ACT ACT GAA AGC GGT GAT AAG  1560
Asp Ser Asp Gln Asp Ala Thr Thr Glu Ser Gly Asp Lys
        510                 515                 520

CCA AAA GAA AAC CTC GAA GAG GAA GAA CAG GAA GCG CAG  1599
Pro Lys Glu Asn Leu Glu Glu Glu Glu Gln Glu Ala Gln
                525                 530

AAT GAG GAA GGA AAG GAA AAA GAA GGC AAT AAA GAT AAA  1638
Asn Glu Glu Gly Lys Glu Lys Glu Gly Asn Lys Asp Lys
    535                 540                 545

GAT GGC GAT GAT GAT AAC GAC AAT GAT GAC GAC GAT GAA  1677
Asp Gly Asp Asp Asp Asn Asp Asn Asp Asp Asp Asp Glu
            550                 555
```

```
GAC GAT CAC AAC TCC GAG GGC GAC GAC GAT GAT GAC GAT  1716
Asp Asp His Asn Ser Glu Gly Asp Asp Asp Asp Asp Asp
560                 565                     570

GAC GAT GAT GAA GAC GAT AAT AAT GAA AAA CAA AGT AAT  1755
Asp Asp Asp Glu Asp Asp Asn Asn Glu Lys Gln Ser Asn
        575                 580                 585

CAA GGC CTC GAC TAC GTC GTA AGG CCG TTT CTG ACA GAG  1794
Gln Gly Leu Asp Tyr Val Val Arg Pro Phe Leu Thr Glu
                590                 595
TAA                                                  1797
```

**SEQ ID No. 3**

Sequence type: Polynucleotide with corresponding polypeptide

Sequence length: 132 base pairs

Strandedness: double

Topology: linear

Immediate experimental source: S. cerevisiae HT246/pJDB207/GAPFL-HIR-CALC

Features: From 1 to 7 linker sequence including PstI restriction site and coding region for linker cleavable by yscF and yscα*, from 8 to 127 coding region for C-terminal glycine precursor of human calcitonin, from 125 to 132 sequence including PstI restriction site

```
G AAG AGG GTA CAG CTG GAT AAA AGA TGT GGT AAC TTG TCT      40
  Lys Arg Val Gln Leu Asp Lys Arg Cys Gly Asn Leu Ser
                      5                   10

ACC TGT ATG TTG GGT ACC TAC ACC CAA GAC TTC AAC AAG        79
Thr Cys Met Leu Gly Thr Thr Thr Gln Asp Phe Asn Lys
     15                  20                  25

TTC CAC ACC TTC CCA CAA ACC GCT ATC GGT GTT GGT GCT       118
Phe His Thr Phe Pro Gln Thr Ala Ile Gly Val Gly Ala
                30                  35

CCA GGT TGA CTGCA                                         132
Pro Gly
 40
```

**SEQ ID No. 4**

Sequence type: Polypeptide

Sequence length: 212 amino acids

Topology: linear

Immediate experimental source: S.cerevisiae AB110/pDP34GAPDH-eglincex-1

Features: From 1 to 71 amino acid sequence of eglin C, from 72 to 79 linker sequence, from 80 to 212 amino acid sequence of the cellulose binding domain of C.fimi Exg protein

```
Met Thr Glu Phe Gly Ser Glu Leu Lys Ser Phe Pro Glu
                    5                       10

Val Val Gly Lys Thr Val Asp Gln Ala Arg Glu Tyr Phe
     15                      20                      25

Thr Leu His Tyr Pro Gln Tyr Asp Val Tyr Phe Leu Pro
               30                      35

Glu Gly Ser Pro Val Thr Leu Asp Leu Arg Tyr Asn Arg
 40                      45                      50

Val Arg Val Phe Tyr Asn Pro Gly Thr Asn Val Val Asn
          55                      60                      65

His Val Pro His Val Gly Lys Arg Glu Ala Glu Ala Trp
                    70                      75

Val Pro Phe Gly Ala Ser Pro Thr Pro Thr Pro Thr Thr
     80                      85                      90

Pro Thr Pro Thr Pro Thr Thr Pro Thr Pro Thr Pro Thr
               95                      100

Ser Gly Pro Ala Gly Cys Gln Val Leu Trp Gly Val Asn
105                     110                     115
```

```
Gln Trp Asn Thr Gly Phe Thr Ala Asn Val Thr Val Lys
        120                 125                 130

Asn Thr Ser Ser Ala Pro Val Asp Gly Trp Thr Leu Thr
                135                 140

Phe Ser Phe Pro Ser Gly Gln Gln Val Thr Gln Ala Trp
    145                 150                 155

Ser Ser Thr Val Thr Gln Ser Gly Ser Ala Val Thr Val
            160                 165

Arg Asn Ala Pro Trp Asn Gly Ser Ile Pro Ala Gly Gly
170                 175                 180

Thr Ala Gln Phe Gly Phe Asn Gly Ser His Thr Gly Thr
        185                 190                 195

Asn Ala Ala Pro Thr Ala Phe Ser Leu Asn Gly Thr Pro
                200                 205

Cys Thr Val Gly
    210
```

## Claims

**Claims for the following Contracting States : AT, BE, CH, DE, DK, FR, GB, IT, LI, LU, NL, SE**

**1.** A method for the production of a biologically active protein comprising treating a fusion protein consisting of

1. one or multiple successive protein segment(s) each consisting of said biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and

2. a polypeptide tag joined to the C-terminal amino acid of said successive protein segment(s),

or consisting of

1. a polypeptide tag joined to the N-terminal amino acid of

2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said linker polypeptide sequence L being joined to said biologically active protein,

with soluble yeast endoprotease yscF and with soluble yeast carboxypeptidase yscα, and isolating said biologically active protein.

2. A method according to claim 1 in which the fusion protein has the formula

$$(P\text{-}L)_m\text{-}T \qquad (I)$$

or

$$T\text{-}(L\text{-}P)_n \qquad (II),$$

in which P is the biologically active protein, L has the meaning given in claim 1, T is a polypeptide tag, m is an integer from 1 to 10 and n is an integer from 2 to 10.

3. A method according to claim 1 in which the fusion protein has the formula

$$(P\text{-}L)_m\text{-}T \qquad (I)$$

in which P, L and T have the meanings given above and m is 1.

4. A method according to claim 1 in which the biologically active protein is of prokaryotic or eukaryotic origin.

5. A method according to claim 1 in which the biologically active protein is of mammalian origin.

6. A method according to claim 1 in which the biologically active protein is a hormone, a polypeptide with immunomodulatory, anti-viral and anti-tumor properties, an antibody, viral antigen, blood clotting factor, a fibrinolytic agent or a growth regulation factor.

7. A method according to claim 1 in which the biologically active protein is selected from the group consisting of a human α interferon, a hybrid interferon, human tissue plasminogen activator, human single chain urokinase type plasminogen activator, a hybrid plasminogen activator, transforming growth factor β, human calcitonin, insulin-like growth factor I and II and desulfatohirudin.

8. A method according to claim 1 in which the linker polypeptide sequence L comprises 2 to about 20 amino acid residues and contains one or multiple pairs of basic amino acids, provided that the N-terminal first and second amino acids as well as the C-terminal penultimate and ultimate amino acids represent such a pair of basic amino acids.

9. A method according to claim 1 in which the linker polypeptide sequence L is a dipeptidyl radical selected from Arg-Arg, Lys-Arg, Lys-Lys and Arg-Lys.

10. A method according to claim 1 in which the polypeptide tag T consists of about 10 to about 1000 amino acid residues.

11. A method according to claim 1 in which m is an integer from 1 to 5.

12. A method according to claim 1 in which n is an integer from 2 to 5.

13. A method according to claim 1 in which soluble yscF is a mutein of yeast endoprotease yscF in which the hydrophobic membrane binding site has been deleted.

14. A method according to claim 1 in which soluble yscα is a mutein of yeast carboxypeptidase yscα in which the hydrophobic membrane binding site has been deleted.

15. A method according to claim 1 which is performed in a buffered solution at pH from about 6.0 to about 7.5 in a temperature range of from about 25°C to about 37°C.

**16.** A method according to claim 1 in which the digestion with soluble yscF is performed in the presence of $Ca^{2+}$ ions.

**17.** A method according to claim 1 in which the fusion protein is treated with a digestive mixture containing both soluble yscF and soluble yscα.

**18.** A method according to claim 1 in which the fusion protein is first treated with soluble yscF and, when the digestion has sufficiently proceeded or is complete, thereupon with soluble yscα.

**19.** A fusion protein consisting of

**1.** one or multiple successive protein segment(s) each consisting of said biologically active protein the C-terminal amino acid of which is joined to a linker polypeptide sequence L the N-terminal first and second and, in the case of multiple successive protein segments, the C-terminal penultimate and ultimate amino acid residues of said linker polypeptide sequence L being basic amino acids selected from Lys and Arg, and

**2.** a polypeptide tag joined to the C-terminal amino acid of said successive protein segment(s),

or consisting of

**1.** a polypeptide tag joined to the N-terminal amino acid of

**2.** multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said linker polypeptide sequence L being joined to said biologically active protein.

**20.** A fusion protein according to claim 19 which is represented by the formula

$$(P\text{-}L)_m\text{-}T \qquad \text{(I)}$$

or

$$T\text{-}(L\text{-}P)_n \qquad \text{(II)},$$

in which P is the biologically active protein, L has the meaning given in claim 19, T is a polypeptide tag, m is an integer from 1 to 10 and n is an integer from 2 to 10.

**21.** An expression vector comprising an expression cassette containing a DNA sequence coding for a fusion protein according to claim 19.

**22.** A host cell transformed with an expression vector according to claim 21.

**23.** A method for the production of a fusion protein according to claim 19 comprising culturing under appropriate nutrient conditions transformed host cells according to claim 22 and isolating said fusion protein.

**Claims for the following Contracting States : ES, GR**

**1.** A method for the production of a biologically active protein comprising treating a fusion protein consisting of

1. one or multiple successive protein segment(s) each consisting of said biologically active protein the C-terminal amino acid of which is joined to a linker polypeptide sequence L the N-terminal first and second and, in the case of multiple successive protein segments, also the C-terminal penultimate and ultimate amino acid residues of said linker polypeptide sequence L being basic amino acids selected from Lys and Arg, and

2. a polypeptide tag joined to the C-terminal amino acid of said successive protein segment(s),

or consisting of

1. a polypeptide tag joined to the N-terminal amino acid of

2. multiple successive protein segments each consisting of a linker polypeptide sequence L the N-terminal first and second as well as the C-terminal penultimate and ultimate amino acid residues of said linker polypeptide sequence L being basic amino acids selected from Lys and Arg and the ultimate basic amino acid of said linker polypeptide sequence L being joined to said biologically active protein,

with soluble yeast endoprotease yscF and with soluble yeast carboxypeptidase yscα, and isolating said biologically active protein.

**2.** A method according to claim 1 in which the fusion protein has the formula

$$(P\text{-}L)_m\text{-}T \qquad (I)$$

or

$$T\text{-}(L\text{-}P)_n \qquad (II),$$

in which P is the biologically active protein, L has the meaning given in claim I, T is a polypeptide tag, m is an integer from 1 to 10 and n is an integer from 2 to 10.

**3.** A method according to claim 1 in which the fusion protein has the formula

$$(P\text{-}L)_m\text{-}T \qquad (I)$$

in which P, L and T have the meanings given above and m is 1.

**4.** A method according to claim 1 in which the biologically active protein is of prokaryotic or eukaryotic origin.

**5.** A method according to claim 1 in which the biologically active protein is of mammalian origin.

**6.** A method according to claim 1 in which the biologically active protein is a hormone, a polypeptide with immunomodulatory, anti-viral and anti-tumor properties, an antibody, viral antigen, blood clotting factor, a fibrinolytic agent or a growth regulation factor.

**7.** A method according to claim 1 in which the biologically active protein is selected from the group consisting of a human α interferon, a hybrid interferon, human tissue plasminogen activator, human single chain urokinase type plasminogen activator, a hybrid plasminogen activator, transforming growth factor β, human calcitonin, insulin-like growth factor I and II and desulfatohirudin.

**8.** A method according to claim 1 in which the linker polypeptide sequence L comprises 2 to about 20 amino acid residues and contains one or multiple pairs of basic amino acids, provided that the N-terminal first and second amino acids as well as the C-terminal penultimate and ultimate amino acids represent such a pair of basic amino acids.

**9.** A method according to claim 1 in which the linker polypeptide sequence L is a dipeptidyl radical selected from Arg-Arg, Lys-Arg, Lys-Lys and Arg-Lys.

**10.** A method according to claim 1 in which the polypeptide tag T consists of about 10 to about 1000 amino acid residues.

**11.** A method according to claim 1 in which m is an integer from 1 to 5.

**12.** A method according to claim 1 in which n is an integer from 2 to 5.

**13.** A method according to claim 1 in which soluble yscF is a mutein of yeast endoprotease yscF in which the hydrophobic membrane binding site has been deleted.

**14.** A method according to claim 1 in which soluble yscα is a mutein of yeast carboxypeptidase yscα in which the hydrophobic membrane binding site has been deleted.

**15.** A method according to claim 1 which is performed in a buffered solution at pH from about 6.0 to about 7.5 in a temperature range of from about 25°C to about 37°C.

**16.** A method according to claim 1 in which the digestion with soluble yscF is performed in the presence of $Ca^{2+}$ ions.

**17.** A method according to claim 1 in which the fusion protein is treated with a digestive mixture containing both soluble yscF and soluble yscα.

**18.** A method according to claim 1 in which the fusion protein is first treated with soluble yscF and, when the digestion has sufficiently proceeded or is complete, thereupon with soluble yscα.

**Patentansprüche**

**Patentansprüche für folgende Vertragsstaaten : AT, BE, CH, DE, DK, FR, GB, IT, LI, LU, NL, SE**

**1.** Verfahren zur Produktion eines biologisch aktiven Proteins, wobei man ein Fusionsprotein, bestehend aus

1. einem oder mehreren aufeinanderfolgenden Proteinsegment(en), jeweils bestehend aus dem biologisch aktiven Protein, dessen C-terminale Aminosäure an eine Linker-Polypeptid-Sequenz L geknüpft ist, wobei die ersten und zweiten N-terminalen und im Falle von mehreren aufeinanderfolgenden Proteinsegmenten auch die vorletzten und letzten C-terminalen Aminosäurereste der Linker-Polypeptid-Sequenz L basische Aminosäuren, ausgewählt aus Lys und Arg, sind und
2. einem Polypeptidende, das an die C-terminale Aminosäure des aufeinanderfolgenden Proteinsegments/der aufeinanderfolgenden Proteinsegmente geknüpft ist,

oder bestehend aus

1. einem Polypeptidende, das an die N-terminale Aminosäure von
2. mehreren aufeinanderfolgenden Proteinsegmenten geknüpft ist, wobei jedes aus einer Linker-Polypeptid-Sequenz L besteht, wobei die ersten und zweiten N-terminalen sowie die vorletzten und letzten C-terminalen Aminosäurereste der Linker-Polypeptid-Sequenz L basische Aminosäuren, ausgewählt aus Lys und Arg, sind, und die letzte basische Aminosäure der Linker-Polypeptid-Sequenz L an das biologisch aktive Protein geknüpft ist,

mit einer löslichen Hefe-Endoprotease yscF und mit einer löslichen Hefe-Carboxypeptidase yscα behandelt, und das biologisch aktive Protein isoliert.

**2.** Verfahren nach Anspruch 1, wobei das Fusionsprotein die Formel

$$(P\text{-}L)_m\text{-}T \qquad (I)$$

oder

$$T\text{-}(L\text{-}P)_n \qquad (II)$$

besitzt, worin P das biologisch aktive Protein ist, L die in Anspruch 1 gegebene Bedeutung besitzt, T ein Polypeptidende ist, m eine ganze Zahl von 1 bis 10 ist, und n eine ganze Zahl von 2 bis 10 ist.

**3.** Verfahren nach Anspruch 1, wobei das Fusionsprotein die Formel

$$(P\text{-}L)_m\text{-}T \qquad (I)$$

besitzt, worin P, L und T, wie vorstehend definiert sind, und m den Wert 1 hat.

**4.** Verfahren nach Anspruch 1, wobei das biologisch aktive Protein prokaryotischen oder eukaryotischen Ursprungs ist.

**5.** Verfahren nach Anspruch 1, wobei das biologisch aktive Protein von einem Säuger stammt.

6. Verfahren nach Anspruch 1, wobei das biologisch aktive Protein ein Hormon, ein Polypeptid mit immunmodulatorischen, antiviralen und Anti-Tumor-Eigenschaften, ein Antikörper, ein virales Antigen, ein Blutgerinnungsfaktor, ein fibrinolytisches Mittel oder ein wachstumsregulierender Faktor ist.

7. Verfahren nach Anspruch 1, wobei das biologisch aktive Protein aus der Gruppe, bestehend aus menschlichem $\alpha$-Interferon, einem Hybridinterferon, menschlichem Gewebsplasminogenaktivator, menschlichem einkettigem Plasminogenaktivator vom Urokinasetyp, einem Hybrid-Plasminogenaktivator, transformierendem Wachstumsfaktor $\beta$, menschlichem Calcitonin, insulinähnlichem Wachstumsfaktor I und II und Desulfatohirudin, ausgewählt wird.

8. Verfahren nach Anspruch 1, wobei die Linker-Polypeptid-Sequenz L 2 bis etwa 20 Aminosäurereste umfaßt und ein oder mehrere Paar(e) basischer Aminosäuren enthält, mit der Maßgabe, daß die ersten und zweiten N-terminalen Aminosäuren sowie die vorletzten und letzten C-terminalen Aminosäuren ein solches Paar basischer Aminosäuren darstellen.

9. Verfahren nach Anspruch 1, wobei die Linker-Polypeptid-Sequenz L ein Dipeptidylrest, ausgewählt aus Arg-Arg, Lys-Arg, Lys-Lys und Arg-Lys, ist.

10. Verfahren nach Anspruch 1, wobei das Polypeptidende T aus etwa 10 bis etwa 1000 Aminosäureresten besteht.

11. Verfahren nach Anspruch 1, wobei m eine ganze Zahl von 1 bis 5 ist.

12. Verfahren nach Anspruch 1, wobei n eine ganze Zahl von 2 bis 5 ist.

13. Verfahren nach Anspruch 1, wobei die lösliche yscF eine Mutante der Hefe-Endoprotease yscF ist, worin die hydrophobe Membranbindungsstelle deletiert wurde.

14. Verfahren nach Anspruch 1, wobei die lösliche ysc$\alpha$ eine Mutante der Hefe-Carboxypeptidase ysc$\alpha$ ist, worin die hydrophobe Membranbindungsstelle deletiert wurde.

15. Verfahren nach Anspruch 1, welches in einer gepufferten Lösung bei einem pH-Wert von etwa 6,0 bis etwa 7,5 in einem Temperaturbereich von etwa 25°C bis etwa 37°C durchgeführt wird.

16. Verfahren nach Anspruch 1, wobei die Spaltung mit löslicher yscF in Gegenwart von $Ca^{2+}$-Ionen durchgeführt wird.

17. Verfahren nach Anspruch 1, wobei das Fusionsprotein mit einem Spaltungsgemisch, enthaltend sowohl lösliche yscF als auch lösliche ysc$\alpha$ behandelt wird.

18. Verfahren nach Anspruch 1, wobei das Fusionsprotein zuerst mit löslicher yscF behandelt wird, und dann, wenn die Spaltung ausreichend fortgeschritten ist oder vollendet ist, anschließend mit löslicher ysc$\alpha$ behandelt wird.

19. Fusionsprotein bestehend aus

1. einem oder mehreren aufeinanderfolgenden Proteinsegment(en), jeweils bestehend aus dem biologisch aktiven Protein, dessen C-terminale Aminosäure an eine Linker-Polypeptid-Sequenz L geknüpft ist, wobei die ersten und zweiten N-terminalen und im Falle von mehreren aufeinanderfolgenden Proteinsegmenten auch die vorletzten und letzten C-terminalen Aminosäurereste der Linker-Polypeptid-Sequenz L basische Aminosäuren, ausgewählt aus Lys und Arg, sind und
2. einem Polypeptidende, das an die C-terminale Aminosäure des aufeinanderfolgenden Proteinsegments/der aufeinanderfolgenden Proteinsegmente geknüpft ist,

oder bestehend aus

1. einem Polypeptidende, das an die N-terminale Aminosäure von
2. mehreren aufeinanderfolgenden Proteinsegmenten geknüpft ist, wobei jedes aus einer Linker-Polypeptid-Sequenz L besteht, wobei die ersten und zweiten N-terminalen sowie die vorletzten und letzten C-terminalen Aminosäurereste der Linker-Polypeptid-Sequenz L basische Aminosäuren, ausgewählt aus Lys und Arg, sind, und die letzte basische Aminosäure der Linker-Polypeptid-Sequenz L an das biologisch aktive Protein geknüpft ist.

**20.** Fusionsprotein nach Anspruch 19, welches durch die Formel

$$(P\text{-}L)_m\text{-}T \qquad (I)$$

oder

$$T\text{-}(L\text{-}P)_n \qquad (II)$$

wiedergeben wird, worin P das biologisch aktive Protein ist, L wie in Anspruch 19 definiert ist, T ein Polypeptidende ist, m eine ganze Zahl von 1 bis 10 ist, und n eine ganze Zahl von 2 bis 10 ist.

**21.** Expressionsvektor, umfassend eine Expressionkassette, enthaltend eine DNA-Sequenz, die ein Fusionsprotein nach Anspruch 19 codiert.

**22.** Mit einem Expressionsvektor nach Anspruch 21 transformierte Wirtszelle.

**23.** Verfahren zur Produktion eines Fusionsproteins nach Anspruch 19, umfassend die Züchtung von transformierten Wirtszellen nach Anspruch 22 unter geeigneten Nährbedingungen und die Isolierung des Fusionsproteins.

**Patentansprüche für folgende Vertragsstaaten : ES, GR**

**1.** Verfahren zur Produktion eines biologisch aktiven Proteins, wobei man ein Fusionsprotein, bestehend aus

1. einem oder mehreren aufeinanderfolgenden Proteinsegment(en), jeweils bestehend aus dem biologisch aktiven Protein, dessen C-terminale Aminosäure an eine Linker-Polypeptid-Sequenz L geknüpft ist, wobei die ersten und zweiten N-terminalen und im Falle von mehreren aufeinanderfolgenden Proteinsegmenten auch die vorletzten und letzten C-terminalen Aminosäurereste der Linker-Polypeptid-Sequenz L basische Aminosäuren, ausgewählt aus Lys und Arg, sind und
2. einem Polypeptidende, das an die C-terminale Aminosäure des aufeinanderfolgenden Proteinsegments/der aufeinanderfolgenden Proteinsegmente geknüpft ist,

oder bestehend aus

1. einem Polypeptidende, das an die N-terminale Aminosäure von
2. mehreren aufeinanderfolgenden Proteinsegmenten geknüpft ist, wobei jedes aus einer Linker-Polypeptid-Sequenz L besteht, wobei die ersten und zweiten N-terminalen sowie die vorletzten und letzten C-terminalen Aminosäurereste der Linker-Polypeptid-Sequenz L basische Aminosäuren, ausgewählt aus Lys und Arg, sind, und die letzte basische Aminosäure der Linker-Polypeptid-Sequenz L an das biologisch aktive Protein geknüpft ist,

mit einer löslichen Hefe-Endoprotease yscF und mit einer löslichen Hefe-Carboxypeptidase ysc$\alpha$ behandelt, und das biologisch aktive Protein isoliert.

**2.** Verfahren nach Anspruch 1, wobei das Fusionsprotein die Formel

$$(P\text{-}L)_m\text{-}T \qquad (I)$$

oder

$$T\text{-}(L\text{-}P)_n \qquad (II)$$

besitzt, worin P das biologisch aktive Protein ist, L die in Anspruch 1 gegebene Bedeutung besitzt, T ein Polypeptidende ist, m eine ganze Zahl von 1 bis 10 ist, und n eine ganze Zahl von 2 bis 10 ist.

**3.** Verfahren nach Anspruch 1, wobei das Fusionsprotein die Formel

$$(P\text{-}L)_m\text{-}T \qquad (I)$$

besitzt, worin P, L und T, wie vorstehend definiert sind, und m den Wert 1 hat.

**4.** Verfahren nach Anspruch 1, wobei das biologisch aktive Protein prokaryotischen oder eukaryotischen Ursprungs ist.

5. Verfahren nach Anspruch 1, wobei das biologisch aktive Protein von einem Säuger abstammt.

6. Verfahren nach Anspruch 1, wobei das biologisch aktive Protein ein Hormon, ein Polypeptid mit immunmodulatorischen, antiviralen und Anti-Tumor-Eigenschaften, ein Antikörper, ein virales Antigen, ein Blutgerinnungsfaktor, ein fibrinolytisches Mittel oder ein wachstumsregulierender Faktor ist.

7. Verfahren nach Anspruch 1, wobei das biologisch aktive Protein aus der Gruppe, bestehend aus menschlichem α-Interferon, einem Hybridinterferon, menschlichem Gewebsplasminogenaktivator, menschlichem einkettigem Plasminogenaktivator vom Urokinasetyp, einem Hybrid-Plasminogenaktivator, transformierendem Wachstumsfaktor β, menschlichem Calcitonin, insulinähnlichem Wachstumsfaktor I und II und Desulfatohirudin, ausgewählt wird.

8. Verfahren nach Anspruch 1, wobei die Linker-Polypeptid-Sequenz L 2 bis etwa 20 Aminosäurereste umfaßt und ein oder mehrere Paar(e) basischer Aminosäuren enthält, mit der Maßgabe, daß die ersten und zweiten N-terminalen Aminosäuren sowie die vorletzten und letzten C-terminalen Aminosäuren ein solches Paar basischer Aminosäuren darstellen.

9. Verfahren nach Anspruch 1, wobei die Linker-Polypeptid-Sequenz L ein Dipeptidylrest, ausgewählt aus Arg-Arg, Lys-Arg, Lys-Lys und Arg-Lys, ist.

10. Verfahren nach Anspruch 1, wobei das Polypeptidende T aus etwa 10 bis etwa 1000 Aminosäureresten besteht.

11. Verfahren nach Anspruch 1, wobei m eine ganze Zahl von 1 bis 5 ist.

12. Verfahren nach Anspruch 1, wobei n eine ganze Zahl von 2 bis 5 ist.

13. Verfahren nach Anspruch 1, wobei die lösliche yscF eine Mutante der Hefe-Endoprotease yscF ist, worin die hydrophobe Membranbindungsstelle deletiert wurde.

14. Verfahren nach Anspruch 1, wobei die lösliche yscα eine Mutante der Hefe-Carboxypeptidase yscα ist, worin die hydrophobe Membranbindungsstelle deletiert wurde.

15. Verfahren nach Anspruch 1, welches in einer gepufferten Lösung bei einem pH-Wert von etwa 6,0 bis etwa 7,5 in einem Temperaturbereich von etwa 25°C bis etwa 37°C durchgeführt wird.

16. Verfahren nach Anspruch 1, wobei die Spaltung mit löslicher yscF in Gegenwart von $Ca^{2+}$-Ionen durchgeführt wird.

17. Verfahren nach Anspruch 1, wobei das Fusionsprotein mit einem Spaltungsgemisch, enthaltend sowohl lösliche yscF als auch lösliche yscα behandelt wird.

18. Verfahren nach Anspruch 1, wobei das Fusionsprotein zuerst mit löslicher yscF behandelt wird, und dann, wenn die Spaltung ausreichend fortgeschritten ist oder vollendet ist, anschließend mit löslicher yscα behandelt wird.

## Revendications

### Revendications pour les Etats contractants suivants : AT, BE, CH, DE, DK, FR, GB, IT, LI, LU, NL, SE

1. Procédé de production d'une protéine biologiquement active comprenant de traiter une protéine fusionnée constituée de :

    1. Un ou plusieurs segment (s) de protéine successifs, chacun étant constitué de ladite protéine biologiquement active dont l'amino-acide C-terminal qui est lié à une séquence L polypeptide de liaison, le premier et le second restes amino-acide N-terminal et, dans le cas de plusieurs segments de protéine successifs aussi l'avant-dernier et le dernier reste amino-acide C-terminal de ladite séquence L polypeptide de liaison étant des amino-acides basiques choisis parmi Lys et Arg, et

2. Un marqueur polypeptide lié à l'amino-acide C-terminal du(des)dit(s) segment(s) de protéine successif(s),

ou constitué de

1. Un marqueur polypeptide lié à l'amino-acide N-terminal de
2. Des segments de protéine successifs multiples, chacun étant constitué d'une séquence L polypeptide de liaison, le premier et le second restes amino-acide N-terminal et l'avant-dernier et le dernier restes amino-acide C-terminal de ladite séquence L polypeptide de liaison étant des amino-acides basiques choisis parmi Lys et Arg, et le dernier amino-acide basique de ladite séquence L polypeptide de liaison étant lié à ladite protéine biologiquement active,

avec l'endoprotéase de levure soluble yscF et avec la carboxypeptidase de levure soluble ysc$\alpha$ et isoler ladite protéine biologiquement active.

**2.** Procédé selon la revendication 1, où la protéine de fusion a la formule

$$(P - L)_m - T \qquad (I)$$

ou

$$T - (L - P)_n \qquad (II),$$

où P est la protéine biologiquement active, L a la signification donnée à la revendication 1, T est un marqueur polypeptide, m est un entier de 1 à 10 et n est un entier de 2 à 10.

**3.** Procédé selon la revendication 1, où la protéine fusionnée a la formule

$$(P - L)_m - T \qquad (I)$$

où P, L et T ont les significations données ci-dessus, et m est 1.

**4.** Procédé selon la revendication 1, où la protéine biologiquement active est d'origine procaryote ou eucaryote.

**5.** Procédé selon la revendication 1, où la protéine biologiquement active est d'origine mammifère.

**6.** Procédé selon la revendication 1 où la protéine biologiquement active est une hormone, un polypeptide avec des propriétés immunomodulatrices, antivirales et antitumeurs, un anticorps, un antigène viral, un facteur de coagulation du sang, un agent fibrinolytique ou un facteur de régulation de croissance.

**7.** Procédé selon la revendication 1, où on choisit la protéine biologiquement active dans le groupe constitué de l'interféron $\alpha$ humain, un interféron hybride, un activateur de plasminogène de tissu humain, un activateur de plasminogène de type urokinase à chaîne unique humain, un activateur de plasminogène hybride, un facteur $\beta$ de croissance transformant, la calcitonine humaine, un facteur I et II de croissance analogue à l'insuline, et la désulfatohirudine.

**8.** Procédé selon la revendication 1, où la séquence L polypeptide de liaison comprend 2 à environ 20 restes amino acides et contient une ou plusieurs paires d'amino acides basiques, à condition que le premier et le second amino acides N-terminaux ainsi que l'avant-dernier et le dernier amino acides C-terminaux représentent une telle paire d'amino acides basiques.

**9.** Procédé selon la revendication 1, où la séquence L polypeptide de liaison est un radical dipeptidyle choisi parmi Arg-Arg, Lys-Arg, Lys-Lys et Arg-Lys.

**10.** Procédé selon la revendication 1, où le marqueur polypeptide T est constitué d'environ 10 à environ 1 000 restes amino acides.

**11.** Procédé selon la revendication 1, où m est un entier de 1 à 5.

**12.** Procédé selon la revendication 1, où n est un entier de 2 à 5.

**13.** Procédé selon la revendication 1, où la yscF soluble est une mutéine d'endoprotéase yscF de levure où on a éliminé le site de fixation sur membrane hydrophobe.

**14.** Procédé selon la revendication 1, où la yscα soluble est une mutéine de carboxypeptidase de levure yscα où on a enlevé le site de fixation sur membrane hydrophobe.

**15.** Procédé selon la revendication 1, où on travaille dans une solution tampon à pH compris entre environ 6,0 et environ 7,5, dans un intervalle de température d'environ 25°C à environ 37°C.

**16.** Procédé selon la revendication 1, où on réalise la digestion avec yscF soluble en présence d'ions $Ca^{2+}$.

**17.** Procédé selon la revendication 1, où on traite la protéine fusionnée avec un mélange de digestion contenant à la fois la yscF soluble et la yscα soluble.

**18.** Procédé selon la revendication 1, où on traite d'abord la protéine fusionnée avec la yscF soluble et, quand la digestion a suffisamment progressé ou est complète, on traite ensuite avec la yscα soluble.

**19.** Protéine fusionnée constituée de :

1. Un ou plusieurs segment(s) de protéine successifs, chacun étant constitué de ladite protéine biologiquement active dont l'amino-acide C-terminal qui est lié à une séquence L polypeptide de liaison, le premier et le second restes amino-acide N-terminal et, dans le cas de plusieurs segments de protéine successifs aussi l'avant-dernier et le dernier reste amino-acide C-terminal de ladite séquence L polypeptide de liaison étant des amino-acides basiques choisis parmi Lys et Arg, et
2. Un marqueur polypeptide lié à l'amino-acide C-terminal du(des)dit(s) segment(s) de protéine successif(s),

ou constitué de

1. Un marqueur polypeptide lié à l'amino-acide N-terminal de
2. Des segments de protéine successifs multiples, chacun étant constitué d'une séquence L polypeptide de liaison, le premier et le second restes amino-acide N-terminal et l'avant-dernier et le dernier restes amino-acide C-terminal de ladite séquence L polypeptide de liaison étant des amino-acides basiques choisis parmi Lys et Arg, et le dernier amino-acide basique de ladite séquence L polypeptide de liaison étant lié à ladite protéine biologiquement active.

**20.** Protéine fusionnée selon la revendication 19, qui est représentée par la formule :

$$(P - L)_m - T \qquad (I)$$

ou

$$T - (L - P)_n \qquad (II),$$

où P est la protéine biologiquement active, L a la signification donnée à la revendication 19, T est un marqueur polypeptide, m est un entier de 1 à 10, et n est un entier de 2 à 10.

**21.** Vecteur d'expression comprenant une cassette d'expression contenant une séquence ADN codant pour une protéine fusionnée selon la revendication 19.

**22.** Cellule hôte transformée avec un vecteur d'expression selon la revendication 21.

**23.** Procédé de production d'une protéine fusionnée selon la revendication 19, comprenant de cultiver dans des conditions nutritives appropriées des cellules hôtes transformées selon la revendication 22, et isoler ladite protéine fusionnée.

**Revendications pour les Etats contractants suivants : ES, GR**

**1.** Procédé de production d'une protéine biologiquement active comprenant de traiter une protéine fusionnée constituée de :

1. Un ou plusieurs segment(s) de protéine successifs, chacun étant constitué de ladite protéine biologiquement active dont l'amino-acide C-terminal qui est lié à une séquence L polypeptide de liaison, le premier et le second restes amino-acide N-terminal et, dans le cas de plusieurs segments de protéine successifs aussi l'avant-dernier et le dernier reste amino-acide C-terminal de ladite séquence L polypeptide de liaison étant des amino-acides basiques choisis parmi Lys et Arg, et
2. Un marqueur polypeptide lié à l'amino-acide C-terminal du(des)dit(s) segment(s) de protéine successif(s),

ou constitué de

1. Un marqueur polypeptide lié à l'amino-acide N-terminal de
2. Des segments de protéine successifs multiples, chacun étant constitué d'une séquence L polypeptide de liaison, le premier et le second restes amino-acide N-terminal et l'avant-dernier et le dernier restes amino-acide C-terminal de ladite séquence L polypeptide de liaison étant des amino-acides basiques choisis parmi Lys et Arg, et le dernier amino-acide basique de ladite séquence L polypeptide de liaison étant lié à ladite protéine biologiquement active,

avec l'endoprotéase de levure soluble yscF et avec la carboxypeptidase de levure soluble ysc$\alpha$ et isoler ladite protéine biologiquement active.

**2.** Procédé selon la revendication 1, où la protéine de fusion a la formule

$$(P - L)_m - T \qquad (I)$$

ou

$$T - (L - P)_n \qquad (II),$$

où P est la protéine biologiquement active, L a la signification donnée à la revendication 1, T est un marqueur polypeptide, m est un entier de 1 à 10 et n est un entier de 2 à 10.

**3.** Procédé selon la revendication 1, où la protéine fusionnée a la formule

$$(P - L)_m - T \qquad (I)$$

où P, L et T ont les significations données ci-dessus, et m est 1.

**4.** Procédé selon la revendication 1, où la protéine biologiquement active est d'origine procaryote ou eucaryote.

**5.** Procédé selon la revendication 1, où la protéine biologiquement active est d'origine mammifère.

**6.** Procédé selon la revendication 1 où la protéine biologiquement active est une hormone, un polypeptide avec des propriétés immunomodulatrices, antivirales et antitumeurs, un anticorps, un antigène viral, un facteur de coagulation du sang, un agent fibrinolytique ou un facteur de régulation de croissance.

**7.** Procédé selon la revendication 1, où on choisit la protéine biologiquement active dans le groupe constitué de l'interféron $\alpha$ humain, un interféron hybride, un activateur de plasminogène de tissu humain, un activateur de plasminogène de type urokinase à chaîne unique humain, un activateur de plasminogène hybride, un facteur $\beta$ de croissance transformant, la calcitonine humaine, un facteur I et II de croissance analogue à l'insuline, et la désulfatohirudine.

**8.** Procédé selon la revendication 1, où la séquence L polypeptide de liaison comprend 2 à environ 20 restes amino acides et contient une ou plusieurs paires d'amino acides basiques, à condition que le premier et le second amino acides N-terminaux ainsi que l'avant-dernier et le dernier amino acides C-terminaux représentent une telle paire d'amino acides basiques.

**9.** Procédé selon la revendication 1, où la séquence L polypeptide de liaison est un radical dipeptidyle choisi parmi Arg-Arg, Lys-Arg, Lys-Lys et Arg-Lys.

**10.** Procédé selon la revendication 1, où le marqueur polypeptideT est constitué d'environ 10 à environ 1 000 restes amino acides.

**11.** Procédé selon la revendication 1, où m est un entier de 1 à 5.

**12.** Procédé selon la revendication 1, où n est un entier de 2 à 5.

**13.** Procédé selon la revendication 1, où la yscF soluble est une mutéine d'endoprotéase yscF de levure où on a éliminé le site de fixation sur membrane hydrophobe.

**14.** Procédé selon la revendication 1, où la yscα soluble est une mutéine de carboxypeptidase de levure yscα où on a enlevé le site de fixation sur membrane hydrophobe.

**15.** Procédé selon la revendication 1, où on travaille dans une solution tampon à pH compris entre environ 6,0 et environ 7,5, dans un intervalle de température d'environ 25°C à environ 37°C.

**16.** Procédé selon la revendication 1, où on réalise la digestion avec yscF soluble en présence d'ions $Ca^{2+}$.

**17.** Procédé selon la revendication 1, où on traite la protéine fusionnée avec un mélange de digestion contenant à la fois la yscF soluble et la yscα soluble.

**18.** Procédé selon la revendication 1, où on traite d'abord la protéine fusionnée avec la yscF soluble et, quand la digestion a suffisamment progressé ou est complète, on traite ensuite avec la yscα soluble.